# EUROPEAN PATENT APPLICATION

(11) **EP 4 559 931 A1**
(43) Date of publication of application: **28.05.2025**
(21) Application number: 23841910.5
(22) Date of filing: 30.05.2023
(51) Int. Cl.: C07K 16/10, C12N 15/13, A61K 39/395, A61K 39/42, A61P 31/14

(54) **ANTI-RESPIRATORY SYNCYTIAL VIRUS NEUTRALIZING ANTIBODY AND USE THEREOF**

(30) Priority: 22.07.2022 CN 202210871810
(71) Applicant: Beijing Wisdomab Biotechnology Co., Ltd, Beijing 101111 (CN); Genrix(Shanghai) Biopharmaceutical Co., Ltd., Shanghai 201203 (CN); Chongqing Genrix Biopharmaceutial Co., Ltd., Chongqing 401319 (CN)
(72) Inventor: FU, Xinlei, Beijing 101103 (CN); LIU, Zhigang, Beijing 100039 (CN); ZHOU, Xiaowei, Beijing 100039 (CN); HAO, Xiaobo, Beijing 102629 (CN); LIU, Yulan, Beijing 100176 (CN); HU, Junjie, Beijing 100176 (CN)
(74) Representative: Nederlandsch Octrooibureau
(86) International application number: PCT/CN2023/097016
(87) International publication number: WO 2024/016842

(57) **Abstract**

Disclosed in the present application are an antibody against respiratory syncytial virus (RSV), a nucleic acid molecule encoding the antibody, an expression vector containing the nucleic acid molecule, a host cell containing the nucleic acid molecule or the vector, and a pharmaceutical composition containing the antibody. Further provided in the present application are a method for preparing and purifying the antibody and the use thereof.

## Description

### CROSS REFERENCE TO RELATED APPLICATION

The present application claims priority to Chinese Patent Application No. 202210871810.9, filed on July 22, 2022, which is incorporated herein by reference in its entirety.

### TECHNICAL FIELD

The present application generally relates to the fields of genetic engineering and antibody medicines. In particular, the present application relates to an antibody against respiratory syncytial virus (RSV) and use of the antibody in the prevention or treatment of a respiratory syncytial virus (RSV) associated disease.

### BACKGROUND

Large-capacity, high-quality antibody libraries provide an important molecule source for screening high-affinity antibodies against any antigen and have high commercial and application value. Currently, most of the high-quality antibody libraries reported in the domestic and overseas are constructed by electrotransformation technique. However, the size of antibody libraries constructed by the electrotransformation technique is limited by the transformation efficiency. The antibody libraries need to be prepared in multiple batches, which is a long period of time and requires a large amount of human and material resources.

Respiratory syncytial virus (RSV) is the most important viral pathogen causing acute lower respiratory tract infections (ALRTI) in children under 5 years of age worldwide¹. RSV infection is the leading cause of hospitalisation for viral respiratory tract infections in infants and young children, and seriously endangers children's health, especially for those born prematurely, with congenital heart disease or primary immunodeficiencies. The top 5 countries with RSV infection incidence are Pakistan, India, Nigeria, China and Indonesia, which contribute nearly half of the global disease burden from RSV-ALRTI².

Respiratory syncytial virus (RSV) belongs to the family Pneumoviridae, the genus Orthopneumovirus, and is non-segmented, single-stranded, negative-sense RNA virus³. It can be classified into two subtypes A and B based on differences in surface antigens⁴. The RSV genome comprises 10 genes encoding 11 proteins. Adhesion protein G and fusion protein F are the main protective antigens on the surface of the virus, which can stimulate the body to produce neutralizing antibody⁵. G protein is highly differentiated between subtypes, so most of the antibodies against G protein are subtype-specific antibodies⁶. F protein is highly conserved between subtypes, and the antibodies induced by F protein can simultaneously inhibit the infection of type A/B RSV viruses.

F protein belongs to the type I transmembrane protein, and its inactive precursor (F0) consists of 574 amino acids⁷. Three F0s form a trimer. The host's furin cleaves F0 between amino acids at positions 109 and 110 and between amino acids at positions 136 and 137 of F0 when it is transported through the Golgi. After cleavage, a short peptide of 27 amino acids in the middle (P27) is released, while the remaining two segments F2 and F1 form an active F protein via disulfide linkages (Cys69-Cys212 and Cys37-Cys439)⁸. A highly hydrophobic fusion peptide (FP) is present at the N-terminus of F1 protein, and is located in the hydrophobic cavity of the protein to avoid being affected by the external hydrophilic environment. When F protein is present on the surface of a virion or a cell, it is not structurally stable, but is in a high-level metastable prefusion glyprotein (Pre-F)⁹. Subsequently, the N-terminus of F1 protein undergoes a series of dramatic structural changes, which induce the occurrence of membrane fusion, thereby leading to viral infection of the cell. At the same time, this process also results in the conversion of F protein from a high-level metastable Pre-F structure to a stable postfusion glyprotein (Post-F).

Now, there is no vaccine for preventing RSV. Palivizumab (trade name: Synagis) is approved by U.S. Food and Drug Administration for the prevention of RSV infection in high-risk infants and young children. It requires up to 5 injections to cover a typical RSV season, which is expensive and cannot be widely applied to a population of infants and young children.

Based on the clinical needs, the development of anti-respiratory syncytial virus antibodies is medically important for preventing diseases associated with RSV infection.

### SUMMARY OF THE INVENTION

In a first aspect, there is provided in the present application an antibody against respiratory syncytial virus (RSV) comprising a heavy chain variable region comprising the amino acid sequences of HCDR1, HCDR2 and HCDR3, and a light chain variable region comprising the amino acid sequences of LCDR1, LCDR2 and LCDR3, wherein
the amino acid sequence of HCDR1 is set forth in SEQ ID NO:32, the amino acid sequence of HCDR2 is set forth in SEQ ID NO:33, the amino acid sequence of HCDR3 is set forth in SEQ ID NO:34, the amino acid sequence of LCDR1 is set forth in SEQ ID NO:35, the amino acid sequence of LCDR2 is set forth in SEQ ID NO:36, and the amino acid sequence of LCDR3 is set forth in SEQ ID NO:37; or
the amino acid sequence of HCDR1 is set forth in SEQ ID NO:38, the amino acid sequence of HCDR2 is set forth in SEQ ID NO:39, the amino acid sequence of HCDR3 is set forth in SEQ ID NO:40, the amino acid sequence of LCDR1 is set forth in SEQ ID NO:41, the amino acid sequence of LCDR2 is set forth in SEQ ID NO:42, and the amino acid sequence of LCDR3 is set forth in SEQ ID NO:43;
wherein the amino acid sequences HCDRs and LCDRs are defined according to Kabat.

In some embodiments of the first aspect, the amino acid sequence of the heavy chain variable region of the antibody is set forth in SEQ ID NO: 28 or 30.

In some embodiments of the first aspect, the amino acid sequence of the light chain variable region of the antibody is set forth in SEQ ID NO: 29 or 31.

In some embodiments of the first aspect, the amino acid sequence of the heavy chain variable region of the antibody is set forth in SEQ ID NO: 28, and the amino acid sequence of the light chain variable region of the antibody is set forth in SEQ ID NO: 29; or
the amino acid sequence of the heavy chain variable region of the antibody is set forth in SEQ ID NO: 30, and the amino acid sequence of the light chain variable region of the antibody is set forth in SEQ ID NO: 31.

In a second aspect, there is provided in the present application an antibody against respiratory syncytial virus (RSV), wherein the amino acid sequence of the heavy chain variable region of the antibody has at least 90% identity to the amino acid sequence as set forth in SEQ ID NO: 28 or 30, and the amino acid sequence of the light chain variable region of the antibody has at least 90% identity to the amino acid sequence as set forth in SEQ ID NO: 29 or 31.

In some embodiments of any one of the first and second aspects, the antibody is a neutralizing antibody.

In some embodiments of any one of the first and second aspects, the antibody can be capable of binding to F protein of human respiratory syncytial virus (RSV).

In some embodiments of any one of the first and second aspects, the antibody is a Fab fragment, a complete antibody, a F(ab')₂ fragment, or a single chain Fv fragment (scFv).

In some embodiments of any one of the first and second aspects, the antibody is a monoclonal antibody.

In some embodiments of any one of the first and second aspects, the antibody comprises a heavy chain constant region of an IgG1 subtype, an IgG2 subtype, or an IgG4 subtype.

In some embodiments of any one of the first and second aspects, the heavy chain constant region comprises a sequence of the Fc fragment of the heavy chain constant region of the IgG1 subtype, and the amino acids at positions 252, 254, 256 of the sequence of the Fc fragment are Y, T, and E, respectively, wherein the amino acid positions of the constant region of the antibody is determined according to EU numbering.

In some embodiments of any one of the first and second aspects, the antibody comprises a light chain constant region of a kappa subtype or a lambda subtype.

In a third aspect, there is provided in the present application a nucleic acid molecule encoding the antibody of the first or second aspect.

In a fourth aspect, there is provided in the present application a pharmaceutical composition comprising the antibody of the first or second aspect and a pharmaceutically acceptable excipient, diluent or carrier.

In a fifth aspect, there is provided in the present application use of the antibody of the first or second aspect, the nucleic acid molecule of the third aspect, or the pharmaceutical composition of the fourth aspect in the manufacture of a medicament for the prevention or treatment of an RSV-associated disease.

In a sixth aspect, there is provided in the present application a method of preventing or treating an RSV-associated disease, comprising administering to a subject in need thereof the antibody of the first or second aspect, or the pharmaceutical composition of the fourth aspect.

In a seventh aspect, there is provided in the present application a plasmid combination for expressing a library of antibody Fab fragments comprising a first plasmid and a second plasmid, wherein
the first plasmid comprises a heavy chain VH-CH1 expression unit of the antibody and a first recombination site;
the second plasmid comprises a light chain VL-CL expression unit of the antibody and a second recombination site; and
the first recombination site is different from the second recombination site.

In some embodiments of the seventh aspect, the plasmid combination comprises a plurality of the first plasmids, and the plurality of the first plasmids carry different heavy chain VH-CH1 expression units of the antibody and have the same first recombination site.

In some embodiments of the seventh aspect, the plasmid combination comprises a plurality of the second plasmids, and the plurality of the second plasmids carry different light chain VL-CL expression units of the antibody and have the same second recombination site.

In some embodiments of the seventh aspect, the first plasmid and/or the second plasmid is a phagemid.

In some embodiments of the seventh aspect, the first plasmid and the second plasmid comprise different replication origins.

In some embodiments of the seventh aspect, the replication origin is selected from the group consisting of one or more of a pBR ori, a CDF ori, a replication origin of M13 filamentous phage (f1 ori), and a p15A ori.

In some embodiments of the seventh aspect, the first recombination site and the second recombination site are sites for phage attachment in each genome of the phage and its host bacteria, respectively.

In some embodiments of the seventh aspect, the first recombination site is attP, and the second recombination site is attB.

In some embodiments of the seventh aspect, the first recombination site and the second recombination site are located at polyclonal enzyme cleavage sites of the first plasmid and the second plasmid, respectively.

In some embodiments of the seventh aspect, the light chain constant region CL of the antibody is a human kappa light chain constant region or a human lambda light chain constant region.

In some embodiments of the seventh aspect, the heavy chain constant region CH1 fragment of the antibody is IgG1, IgG2, IgG3, or IgG4 subtype.

In some embodiments of the seventh aspect, the first plasmid and/or the second plasmid comprises a resistance gene coding region.

In some embodiments of the seventh aspect, a nucleic acid molecule comprising a nucleic acid molecule encoding the heavy chain constant region CH1 fragment of the antibody is fused at its 3' end to the 5' end of a nucleic acid molecule encoding protein gIII of filamentous phage M13.

In some embodiments of the seventh aspect, the resistance gene is an antibiotic resistance gene.

In some embodiments of the seventh aspect, the resistance gene is selected from the group consisting of a chloramphenicol resistance gene (CmR), an ampicillin resistance gene (Ampr), a kanamycin resistance gene (KaR), and a tetracycline resistance gene (TetR).

In an eighth aspect, there is provided in the present application a recombinant system for expressing a library of antibody Fab fragments comprising a first plasmid, a second plasmid, and a cell expressing a phage integrase; wherein
the first plasmid comprises a heavy chain VH-CH1 expression unit of the antibody and a first recombination site;
the second plasmid comprises a light chain VL-CL expression unit of the antibody and a second recombination site; and
the first recombination site is different from the second recombination site.

In some embodiments of the eighth aspect, the recombinant system comprises a plurality of the first plasmids, and the plurality of the first plasmids carry different heavy chain VH-CH1 expression units of the antibody and have the same first recombinant site.

In some embodiments of the eighth aspect, the recombinant system comprises a plurality of the second plasmids, and the plurality of the second plasmids carry different light chain VL-CL expression units of the antibody and have the same second recombinant site.

In some embodiments of the eighth aspect, the first plasmid and/or the second plasmid is a phagemid.

In some embodiments of the eighth aspect, the first plasmid and the second plasmid comprise different replication origins.

In some embodiments of the eighth aspect, the replication origin is selected from the group consisting of one or more of a pBR ori, a CDF ori, a replication origin of M13 filamentous phage (f1 ori), and a p15A ori.

In some embodiments of the eighth aspect, the first recombination site and the second recombination site are sites for phage attachment in each genome of the phage and its host bacteria, respectively.

In some embodiments of the eighth aspect, the first recombination site is attP, and the second recombination site is attB.

In some embodiments of the eighth aspect, the first recombination site and the second recombination site are located at polyclonal enzyme cleavage sites of the first plasmid and the second plasmid, respectively.

In some embodiments of the eighth aspect, the light chain constant region CL of the antibody is a human kappa light chain constant region or a human lambda light chain constant region.

In some embodiments of the eighth aspect, the heavy chain constant region CH1 fragment of the antibody is IgG1, IgG2, IgG3, or IgG4 subtype.

In some embodiments of the eighth aspect, the first plasmid and/or the second plasmid comprises a resistance gene coding region.

In some embodiments of the eighth aspect, a nucleic acid molecule comprising a nucleic acid molecule encoding the heavy chain constant region CH1 fragment of the antibody is fused at its 3' end to the 5' end of the nucleic acid molecule encoding protein gIII of filamentous phage M13.

In some embodiments of the eighth aspect, the resistance gene is an antibiotic resistance gene.

In some embodiments of the eighth aspect, the resistance gene is selected from the group consisting of a chloramphenicol resistance gene (CmR), an ampicillin resistance gene (Ampr), a kanamycin resistance gene (KaR), and a tetracycline resistance gene (TetR).

In some embodiments of the eighth aspect, the phage integrase is a tyrosine integrase.

In some embodiments of the eighth aspect, the phage integrase is a lambda phage integrase.

In some embodiments of the eighth aspect, the phage integrase is an inducibly expressed or constitutively expressed integrase.

In some embodiments of the eighth aspect, the phage integrase is an inducibly expressed integrase.

In some embodiments of the eighth aspect, the cell expressing the phage integrase is a prokaryotic cell.

In some embodiments of the eighth aspect, the cell expressing the phage integrase is *E*. *coli.*

In some embodiments of the eighth aspect, the cell expressing the phage integrase is a genetically engineered bacterium.

In some embodiments of the eighth aspect, the first plasmid, the second plasmid, and the cell expressing the phage integrase exist independently of each other, or at least one of the first plasmid and the second plasmid has been introduced into the cell expressing the phage integrase.

In a ninth aspect, there is provided in the present application a recombinant cell for expressing a library of antibody Fab fragments comprising a first plasmid, a second plasmid and expressing a phage integrase; wherein
the first plasmid comprises a heavy chain VH-CH1 expression unit of the antibody and a first recombination site;
the second plasmid comprises a light chain VL-CL expression unit of the antibody and a second recombination site; and
the first recombination site is different from the second recombination site.

In some embodiments of the ninth aspect, the recombinant cell comprises a plurality of the first plasmids, and the plurality of the first plasmids carry different heavy chain VH-CH1 expression units of the antibody and have the same first recombination site.

In some embodiments of the ninth aspect, the recombinant cell comprises a plurality of the second plasmids, and the plurality of the second plasmids carry different light chain VL-CL expression units of the antibody and have the same second recombination site.

In some embodiments of the ninth aspect, the first plasmid and/or the second plasmid is a phagemid.

In some embodiments of the ninth aspect, the first plasmid and the second plasmid comprise different replication origins.

In some embodiments of the ninth aspect, the replication origin is selected from the group consisting of one or more of a pBR ori, a CDF ori, a replication origin of M13 filamentous phage (f1 ori), and a p15A ori.

In some embodiments of the ninth aspect, the first recombination site and the second recombination site are sites for phage attachment in each genome of the phage and its host bacteria, respectively.

In some embodiments of the ninth aspect, the first recombination site is attP, and the second recombination site is attB.

In some embodiments of the ninth aspect, the first recombination site and the second recombination site are located at polyclonal enzyme cleavage sites of the first plasmid and the second plasmid, respectively.

In some embodiments of the ninth aspect, the light chain constant region CL of the antibody is a human kappa light chain constant region or a human lambda light chain constant region.

In some embodiments of the ninth aspect, the heavy chain constant region CH1 fragment of the antibody is IgG1, IgG2, IgG3, or IgG4 subtype.

In some embodiments of the ninth aspect, the first plasmid and/or the second plasmid comprises a resistance gene coding region.

In some embodiments of the ninth aspect, a nucleic acid molecule comprising a nucleic acid molecule encoding the heavy chain constant region CH1 fragment of the antibody is fused at its 3' end to the 5' end of the nucleic acid molecule encoding protein gIII of filamentous phage M13.

In some embodiments of the ninth aspect, the resistance gene is an antibiotic resistance gene.

In some embodiments of the ninth aspect, the resistance gene is selected from the group consisting of a chloramphenicol resistance gene (CmR), an ampicillin resistance gene (Ampr), a kanamycin resistance gene (KaR), and a tetracycline resistance gene (TetR).

In some embodiments of the ninth aspect, the phage integrase is a tyrosine integrase.

In some embodiments of the ninth aspect, the phage integrase is a lambda phage integrase.

In some embodiments of the ninth aspect, the phage integrase is an inducibly expressed or constitutively expressed integrase.

In some embodiments of the ninth aspect, the phage integrase is an inducibly expressed integrase.

In some embodiments of the ninth aspect, the recombinant cell is a prokaryotic cell.

In some embodiments of the ninth aspect, the recombinant cell is *E*. *coli.*

In some embodiments of the ninth aspect, the recombinant cell is a genetically engineered bacterium.

In a tenth aspect, there is provided in the present application a method of preparing a library of antibody Fab fragments, comprising transducing a first plasmid and a second plasmid into a cell expressing a phage integrase; wherein
the first plasmid comprises a heavy chain VH-CH1 expression unit of the antibody and a first recombination site;
the second plasmid comprises a light chain VL-CL expression unit of the antibody and a second recombination site; and
the first recombination site is different from the second recombination site.

In some embodiments of the tenth aspect, a plurality of the first plasmids are transduced into the cell expressing the phage integrase, wherein the plurality of the first plasmids carry different heavy chain VH-CH1 expression units of the antibody and have the same first recombination site.

In some embodiments of the tenth aspect, a plurality of the second plasmids are transduced into the cell expressing the phage integrase, wherein the plurality of the second plasmids carry different light chain VL-CL expression units of the antibody and have the same second recombination site.

In some embodiments of the tenth aspect, transducing the first plasmid and/or the second plasmid into the cell expressing the phage integrase comprises the steps of:
(1) transducing the first plasmid and/or the second plasmid into a competent cell;
(2) infecting the competent cell obtained in step (1) with a helper phage to construct a phage library; and
(3) Transducing the phage library obtained in step (2) into the cell expressing the phage integrase.

In some embodiments of the tenth aspect, the competent cell is a prokaryotic cell.

In some embodiments of the tenth aspect, the competent cell does not express a phage integrase.

In some embodiments of the tenth aspect, the helper phage is an M13 helper phage.

In some embodiments of the tenth aspect, the first plasmid and/or the second plasmid are (is) a phagemid.

In some embodiments of the tenth aspect, the first plasmid and the second plasmid comprise different replication origins.

In some embodiments of the tenth aspect, the replication origin is selected from the group consisting of one or more of a pBR ori, a CDF ori, a replication origin of M13 filamentous phage (f1 ori), and a p15A ori.

In some embodiments of the tenth aspect, the first recombination site and the second recombination site are sites for phage attachment in each genome of the phage and its host bacteria, respectively.

In some embodiments of the tenth aspect, the first recombination site is attP, the second recombination site is attB.

In some embodiments of the tenth aspect, the first recombination site and the second recombination site are located at polyclonal enzyme cleavage sites of the first plasmid and the second plasmid, respectively.

In some embodiments of the tenth aspect, the light chain constant region CL of the antibody is a human kappa light chain constant region or a human lambda light chain constant region.

In some embodiments of the tenth aspect, the heavy chain constant region CH1 fragment of the antibody is IgG1, IgG2, IgG3, or IgG4 subtype.

In some embodiments of the tenth aspect, the first plasmid and/or the second plasmid comprises a resistance gene coding region.

In some embodiments of the tenth aspect, the resistance gene is selected from the group consisting of a chloramphenicol resistance gene (CmR), an ampicillin resistance gene (Ampr), a kanamycin resistance gene (KaR), and a tetracycline resistance gene (TetR).

In some embodiments of the tenth aspect, the phage integrase is a tyrosine integrase.

In some embodiments of the tenth aspect, the phage integrase is a lambda phage integrase.

In some embodiments of the tenth aspect, the phage integrase is an inducibly expressed or constitutively expressed integrase.

In some embodiments of the tenth aspect, the phage integrase is an inducibly expressed integrase.

In some embodiments of the tenth aspect, the cell expressing the phage integrase is a prokaryotic cell.

In some embodiments of the tenth aspect, the cell expressing the phage integrase is *E*. *coli.*

In some embodiments of the tenth aspect, the cell expressing the phage integrase is a genetically engineered bacterium.

In an eleventh aspect, there is provided in the present application use of the plasmid combination of the seventh aspect, the recombinant system of the eighth aspect, or the recombinant cell of the ninth aspect in the manufacture of a library of antibody Fab fragments.

### DESCRIPTION OF THE DRAWINGS

Figure 1 shows a schematic structural diagram of the phagemid vector pHGDisn-attP-new.
Figure 2 shows a schematic structural diagram of the prokaryotic expression vector pHKb-attB-new.
Figure 3 shows the results of ELISA assessing the binding activity of the recombinant monoclonal antibodies against F protein of RSV (R3B1h1, R22B1 and the positive control antibodies) to RSV-DS-Cav1-A (Panel A) and RSV-DS-Cav1-B (Panel B).
Figure 4 shows the results of ELISA assessing the ability of the monoclonal antibodies against F protein of RSV (R3B1h1, R22B1 and Clesrovimab) to block the binding of the phages against F protein of RSV to RSV-DS-Cav1-B; where Panels A-C represent the results of the ability of R3B1h1, R22B1 and Clesrovimab to block the binding of the purified phage against F protein of RSV to RSV-DS-Cav1-B, respectively.
Figure 5 shows the results of a Rapid Fluorescent Focus Inhibition Test (RFFIT) detecting the ability of the monoclonal antibodies against F protein of RSV to inhibit infection of Hep-2 cells by a RSV/A2 strain.
Figure 6 shows the results of RFFIT detecting the ability of the monoclonal antibodies against F protein of RSV to inhibit the infection of Hep-2 cells by a RSV/18537 strain.
Figure 7 shows the results of the monoclonal antibodies against F protein of RSV inhibiting the infection of Hep-2 cells by RSV isolated from clinical samples.

### DESCRIPTION OF THE SEQUENCES

SEQ ID NO:1 shows the nucleotide sequence derived from *Escherichia coli,* encoding a promoter of a chloramphenicol resistance gene (Cat promoter).
SEQ ID NO:2 shows the nucleotide sequence derived from *Lambda phage targeting Escherichia,* encoding the attP attachment site.
SEQ ID NO:3 shows the nucleotide sequence derived from *E. coli,* encoding CDF ori.
SEQ ID NO:4 shows the nucleotide sequence derived from *E. coli,* encoding the attB attachment site.
SEQ ID NO:5 shows the nucleotide sequence derived from *E*. *coli,* encoding the chloramphenicol resistance gene.
SEQ ID NO:6 shows the nucleotide sequence derived from *E. coli,* encoding the complete ampicillin resistance gene expression element.
SEQ ID NO:7 shows the nucleotide sequence derived from *Lambda phage targeting Escherichia,* encoding a lactose manipulator inducible lambda phage integrase expression element.
SEQ ID NO:8 shows the nucleotide sequence encoding the attL recombination site.
SEQ ID NO:9 shows the nucleotide sequence encoding the attR recombination site.
SEQ ID NO: 10 shows the amino acid sequence of F protein of strain A2 of subtype A RSV.
SEQ ID NO:11 shows the amino acid sequence of F protein of strain 18537 of subtype B RSV.
SEQ ID NO: 12 shows the amino acid sequence of F protein mutant RSV-DS-Cav1-A with a DS-Cav1 structure before fusion.
SEQ ID NO: 13 shows the amino acid sequence of F protein mutant RSV-DS-Cav1-B with a DS-Cav1 structure before fusion.
SEQ ID NO: 14 shows the amino acid sequence of the His tag.
SEQ ID NO:15 shows the amino acid sequence of the heavy chain constant region of the human (*homo sapiens*) IgG1 subtype.
SEQ ID NO:16 shows the amino acid sequence of the heavy chain constant region mutant IgG1-YTE of the human (*Homo sapiens*) IgG1 subtype.
SEQ ID NO: 17 shows the amino acid sequence of the heavy chain constant region of the murine (*Mus musculus*) IgG2a subtype.
SEQ ID NO:18 shows the amino acid sequence of the light chain constant region of the human (*Homo sapiens*) kappa subtype.
SEQ ID NO:19 shows the amino acid sequence of the light chain constant region of the human (*Homo sapiens*) lambda subtype.
SEQ ID NO:20 shows the amino acid sequence of the light chain constant region of the murine (*Mus musculus*) kappa subtype.
SEQ ID NO:21 shows the amino acid sequence of the light chain constant region of the murine (*Mus musculus*) lambda subtype.
SEQ ID NO:22 shows the amino acid sequence of the heavy chain variable region of the humanized anti-RSV monoclonal antibody Palivizumab (Hu1129).
SEQ ID NO:23 shows the amino acid sequence of the light chain variable region of the humanized anti-RSV monoclonal antibody Palivizumab (Hu1129).
SEQ ID NO:24 shows the amino acid sequence of the heavy chain variable region of the fully human anti-RSV monoclonal antibody Nirsevimab (MEDI8897).
SEQ ID NO:25 shows the amino acid sequence of the light chain variable region of the fully human anti-RSV monoclonal antibody Nirsevimab (MEDI8897).
SEQ ID NO:26 shows the amino acid sequence of the heavy chain variable region of the fully human anti-RSV monoclonal antibody Clesrovimab (RB1).
SEQ ID NO:27 shows the amino acid sequence of the light chain variable region of the fully human anti-RSV monoclonal antibody Clesrovimab (RB1).
SEQ ID NO:28 shows the amino acid sequence of the heavy chain variable region of the Fab antibody R3B1h1 against F protein of RSV.
SEQ ID NO:29 shows the amino acid sequence of the light chain variable region of the Fab antibody R3B1h1 against F protein of RSV.
SEQ ID NO:30 shows the amino acid sequence of the heavy chain variable region of the Fab antibody R22B1 against F protein of RSV.
SEQ ID NO:31 shows the amino acid sequence of the light chain variable region of the Fab antibody R22B1 against F protein of RSV.
SEQ ID NO:32-34 shows the amino acid sequences of HCDR1, HCDR2 and HCDR3 of the heavy chain variable region of the Fab antibody R3B1h1 against F protein of RSV, respectively.
SEQ ID NO:35-37 shows the amino acid sequences of LCDR1, LCDR2 and LCDR3 of the light chain variable region of the Fab antibody R3B1h1 against F protein of RSV, respectively.
SEQ ID NO:38-40 shows the amino acid sequences of HCDR1, HCDR2 and HCDR3 of the heavy chain variable regions of the Fab antibody R22B1 against F protein of RSV, respectively.
SEQ ID NO:41-43 shows the amino acid sequences of LCDR1, LCDR2 and LCDR3 of the light chain variable regions of the Fab antibody R22B1 against F protein of RSV, respectively.
SEQ ID NO:44 shows the nucleotide sequence derived from *E. coli,* encoding pBR ori.
SEQ ID NO:45 shows the nucleotide sequence derived from f1 phage, encoding f1 ori.
SEQ ID NO:46 shows the nucleotide sequence derived from M13 phage, encoding protein gIII of M13 filamentous phage.

### DETAILED DESCRIPTION OF THE INVENTION

Specific recombinant integration and cleavage of target nucleotide sequence *in vivo* and *in vitro* can be achieved by genetic engineering means using a specific recombinant system or recombinant cell with lambda phage integrase (Int). It has been reported in the literature that specific recombination of a light chain expression plasmid of an antibody and a heavy chain expression plasmids of an antibody in cells can be achieved using genetically engineered bacteria expressing Int integrase by heat induction, which produces a complete plasmid expressing functional Fab¹⁰. Using the Gateway homologous recombination technique of Thermo Fisher which utilizes the specific recombination principle of lambda phage integrase, the efficiency of *in vitro* recombination can be up to 95% without the need for classical gene cloning steps such as digestion and ligation. This technique is used to prepare a Gateway kit for the construction of a cDNA library with a diversity of 1.0E+7.

The present application overcomes the disadvantage of insufficient DNA transformation efficiency during construction of an antibody library by efficient infection of the phage using the recombination system or the recombinant cell with lambda phage integrase. By Infection with, such as heavy chain VH-CH1 expression unit, can induce *E.coli* with plasmids that express Int integrase and contain the light chain expression unit of the antibody, is realized by, and a large capacity Fab antibody library (more than 1.0E+12) is obtained by specific recombination of a heavy chain expression unit (such as a heavy chain VH-CH1 expression unit) of an antibody and a light chain expression unit of an antibody using Int integrase *in vivo* and antibiotic screening using a phage packaged with the heavy chain expression unit (such as the heavy chain VH-CH1 expression unit) of the antibody to infect *E.coli* that can induce expression of Int integrase and comprises a plasmid containing a light chain expression unit of the antibody.

The inventors of the present application utilized the constructed Fab antibody library described above to screen for new Fab antibodies against RSV. In various aspects of the present application, there are provided a new antibody against RSV, a nucleic acid molecule encoding the antibody, a vectors comprising the nucleic acid molecule, a host cell comprising the nucleic acid molecule or the vector, a composition comprising the antibody, a method for preparing and purifying the antibody, and medical and biological applications of the antibody. According to the amino acid sequences of the variable regions of the antibody provided in the present application, a full-length antibody molecule can be constructed as a medicament for preventing or treating RSV-associated diseases.

Unless otherwise indicated, the inventions of the present application can be implemented using conventional molecular biology, microbiology, cell biology, biochemistry, and immunological techniques in the art.

Unless otherwise indicated, the terms used in the present application have the meanings commonly understood by those skilled in the art.

### DEFINITIONS

As used herein, the term "recombination site" refers to a site for phage integration in a phage genome and a bacterial genome. In some embodiments of the present application, the first recombination site and the second recombination site can be a site for phage attachment in each genome of the phage and its host bacteria, respectively, including the case where the first recombination site is a site for phage attachment in the phage genome and the second recombination site is a site for phage attachment in the genome of the host bacteria of the phage; or where the second recombination site is a site for phage attachment in the phage genome and the first recombination site is a site for phage attachment in the genome of the host bacterium of the phage.

As used herein, the term "plasmid" refers to a DNA molecule other than a chromosome (or nucleoid) in an organism such as bacteria, yeast, and actinomycete, and the like. It is a closed-loop double-stranded DNA molecule, presenting in the cytoplasm (with the exception of yeast, where the 2µm plasmid of yeast is present in the nucleus), having the ability to autonomously replicate so that a constant copy number is also maintained in the progeny, and expressing the genetic information it carries. As used herein, the term "plasmid" encompasses a bacterial plasmid, a phagemid, and the like. A "bacterial plasmid" is a vector commonly used in DNA recombination techniques, and is a tool for delivering a useful exogenous gene into a recipient cell for proliferation and expression by genetically engineered means. A "phagemid" is a vector formed by combining a plasmid vector and a single-stranded phage vector, containing a single-stranded phage packaging sequence, a replicon, a plasmid replicon, a cloning site, a marker gene, and the like. In host cells such as *E. coli,* it can replicate as normal double-stranded plasmid DNA molecules, while in the presence of helper phages, it can replicate in a rolling-loop model to produce a single-stranded DNA which can be packaged into a phage particle.

As used herein, the term "attP attachment site" refers to a specific sequence in the phage DNA containing a core region of 15bp which having a sequence identical to the corresponding sequence of the host chromosome.

As used herein, the term "attB attachment site" refers to an attachment site in the bacterial genome containing a core region of15bp which having a sequence identical to the corresponding sequence of the phage genome.

As used herein, the term "phage integrase" refers to a protein with type I topoisomerase activity that specifically binds to attP and attB attachment sites and is indispensable for the integration of phage into the host genome by site-specific recombination.

As used herein, the term "replication origin" refers to an origin site for the plasmid replication, which consists of the origin replicon (ORI) and its regulatory element.

As used herein, the term "antibody" refers to an immunoglobulin molecule that is capable of specifically binding to a target via at least one antigen recognition site located in a variable region of the immunoglobulin molecule. Targets include, but are not limited to, carbohydrates, polynucleotides, lipids, and polypeptides, etc. As used herein, an "antibody" includes not only an intact (*i.e.,* full-length) antibody, but also an antigen-binding fragment thereof (*e.g.,* Fab, Fab', F(ab')₂, or Fv), a variant thereof, a fusion protein comprising portions of an antibody, a humanized antibody, a chimeric antibody, a diabody, a linear antibody, a single-chain fragment variable, a multi-specific antibody (*e*.*g*., a bispecific antibody), and any other modified configurations of an immunoglobulin molecule comprising a desired specific antigen recognition site, including a glycosylated variant of an antibody, an amino acid sequence variant of an antibody, and a covalently modified antibody.

Typically, an intact or full-length antibody comprises two heavy chains and two light chains. Each heavy chain contains a heavy chain variable region (VH) and first, second and third constant regions (CH1, CH2 and CH3). Each light chain contains a light chain variable region (VL) and a constant region (CL). A full-length antibody may be of any type of antibody, such as an IgD, IgE, IgG, IgA, or IgM antibody (or a subtype thereof), but not necessarily belongs to any particular type. Immunoglobulins can be assigned to different types depending on their amino acid sequences of the heavy chain constant domains. Generally, immunoglobulins have five main types, *i.e.,* IgA, IgD, IgE, IgG, and IgM, and some of these types can be further classified into subtypes (isotypes), such as IgG1, IgG2, IgG3, IgG4, IgA1, and IgA2. Heavy chain constant domains corresponding to individual immunoglobulin types are referred to as α, δ, ε, γ, and µ, respectively. Subunit structures and three-dimensional structures of different types of immunoglobulins are well known.

As used herein, the term "antigen binding fragment" or "antigen-binding portion" refers to a portion or region of an intact antibody molecule responsible for binding an antigen. An antigen binding domain can comprise a heavy chain variable region (VH), a light chain variable region (VL), or both. Each of VH and VL typically contains three complementarity determining regions, *i.e.,* CDR1, CDR2, and CDR3.

It is well known to those skilled in the art that complementarity determining regions (CDRs, usually including CDR1, CDR2 and CDR3) are the regions of a variable region that have mostly impact on the affinity and specificity of an antibody. The CDR amino acid sequences of VH or VL have two common definitions, *i.e*., the Chothia definition and the Kabat definition¹¹⁻¹³. For the amino acid sequences of the variable regions of a given antibody, the CDR amino acid sequences in VH and VL amino acid sequences can be determined according to the Chothia definition or the Kabat definition. In an embodiment of the present application, the CDR amino acid sequences are defined according to Kabat.

For the amino acid sequences of the variable regions of a given antibody, the CDR amino acid sequences in the amino acid sequences of variable regions can be determined in a variety of ways, for example, using online software Abysis (http://www.abysis.org/).

Examples of an antigen-binding fragment include, but are not limited to, (1) an Fab fragment, which can be a monovalent fragment having a VL-CL chain and a VH-CH1 chain; (2) an F(ab')₂ fragment, which can be a divalent fragment having two Fab' fragments linked by a disulfide bridge of the hinge region (*i.e.,* a dimer of Fab'); (3) an Fv fragment having VL and VH domains in a single arm of an antibody; (4) a single-chain Fv (scFv), which can be a single polypeptide chain consisting of a VH domain and a VL domain linked by a polypeptide linker; and (5) (scFv)₂, which can comprise two VH domains linked by a peptide linker and two VL domains that are combined with the two VH domains via a disulfide bridge.

In some specific embodiments of the present application, the antibody is a Fab fragment, *i.e.,* a monovalent fragment having a VL-CL chain and a VH-CH1 chain.

As used herein, the term "Fd fragment" refers to a fragment consisting of the heavy chain variable region VH of an antibody and the first constant region CH1 of the heavy chain of the antibody.

As used herein, the term "specific binding" refers to a non-random binding reaction between two molecules, *e.g*., binding of an antibody to an antigen epitope.

As used herein, the term "neutralizing antibody" refers to an antibody that is capable of binding to an antigen on the surface of a pathogenic microorganism, thereby preventing invasion of a cell by preventing the pathogenic microorganism from adhering to a target cell receptor, or preventing the viral envelope from fusing with a cell membrane.

As used herein, the term "monoclonal antibody" refers to an antibody obtained from a substantially homogeneous population of antibodies, *i.e.,* the individual antibodies constituting the population are the same except for naturally occurring mutations that may be present in a small number of individuals.

In a first aspect, there is provided in the present application an antibody against respiratory syncytial virus (RSV) comprising a heavy chain variable region comprising the amino acid sequences of HCDR1, HCDR2 and HCDR3, and a light chain variable region comprising the amino acid sequences of LCDR1, LCDR2 and LCDR3, wherein
the amino acid sequence of HCDR1 is set forth in SEQ ID NO:32, the amino acid sequence of HCDR2 is set forth in SEQ ID NO:33, the amino acid sequence of HCDR3 is set forth in SEQ ID NO:34, the amino acid sequence of LCDR1 is set forth in SEQ ID NO:35, the amino acid sequence of LCDR2 is set forth in SEQ ID NO:36, and the amino acid sequence of LCDR3 is set forth in SEQ ID NO:37; or
the amino acid sequence of HCDR1 is set forth in SEQ ID NO:38, the amino acid sequence of HCDR2 is set forth in SEQ ID NO:39, the amino acid sequence of HCDR3 is set forth in SEQ ID NO:40, the amino acid sequence of LCDR1 is set forth in SEQ ID NO:41, the amino acid sequence of LCDR2 is set forth in SEQ ID NO:42, and the amino acid sequence of LCDR3 is set forth in SEQ ID NO:43;
wherein the amino acid sequences of HCDRs and LCDRs are defined according to Kabat.

In some embodiments of the first aspect, the amino acid sequence of the heavy chain variable region of the antibody is set forth in SEQ ID NO: 28 or 30.

In some embodiments of the first aspect, the amino acid sequence of the light chain variable region of the antibody is set forth in SEQ ID NO: 29 or 31.

In some embodiments of the first aspect, the amino acid sequence of the heavy chain variable region of the antibody is set forth in SEQ ID NO: 28, and the amino acid sequence of the light chain variable region of the antibody is set forth in SEQ ID NO: 29; or
the amino acid sequence of the heavy chain variable region of the antibody is set forth in SEQ ID NO: 30, and the amino acid sequence of the light chain variable region of the antibody is set forth in SEQ ID NO: 31.

In a second aspect, there is provided in the present application an antibody against respiratory syncytial virus (RSV), wherein the amino acid sequence of the heavy chain variable region of the antibody has at least 90% identity to the amino acid sequence as set forth in SEQ ID NO: 28 or 30, and the amino acid sequence of the light chain variable region of the antibody has at least 90% identity to the amino acid sequence as set forth in SEQ ID NO: 29 or 31.

In some embodiments of the second aspect, the amino acid sequence of the heavy chain variable region of the antibody has at least 90%, 91%, 92%, 93%, 94%, 95%, 96%, 97%, 98%, 99% or more identity to the amino acid sequence as set forth in SEQ ID NO: 28 or 30.

In some embodiments of the second aspect, the amino acid sequence of the light chain variable region of the antibody has at least 90%, 91%, 92%, 93%, 94%, 95%, 96%, 97%, 98%, 99% or more identity to the amino acid sequence as set forth in SEQ ID NO: 29 or 31.

In some embodiments of the second aspect, the amino acid sequence of the heavy chain variable region of the antibody differs from the amino acid sequence as set forth in any one of SEQ ID NOs: 28 and 30 by about 1, 2, 3, 4, 5, 6, 7, 8, 9, or 10 amino acid substitutions, deletions, and/or additions.

In some embodiments of the second aspect, the amino acid sequence of the light chain variable region of the antibody differs from the amino acid sequence as set forth in any one of SEQ ID NOs: 29 and 31 by about 1, 2, 3, 4, 5, 6, 7, 8, 9, or 10 amino acid substitutions, deletions, and/or additions.

In some embodiments of the second aspect, the C-terminal or N-terminal region of the amino acid sequence as set forth in any one of SEQ ID NOs: 28 and 30 can also be truncated by about 1, 2, 3, 4, 5, 6, 7, 8, 9, 10, 11, 12, 13, 14, 15, 20, 25, or more amino acids, while the resulting amino acid sequences still retain the function similar to that of the heavy chain variable region of the antibody.

In some embodiments of the second aspect, 1, 2, 3, 4, 5, 6, 7, 8, 9, 10, 11, 12, 13, 14, 15, 20, 25, or more amino acids can also be added to the C-terminal or N-terminal region of the amino acid sequence as set forth in any one of SEQ ID NOs: 28 and 30, while the resulting amino acid sequences still retain the function similar to that of the heavy chain variable region of the antibody.

In some embodiments of the second aspect, 1, 2, 3, 4, 5, 6, 7, 8, 9, 10, 11, 12, 13, 14, 15, 20, 25, or more amino acids can also be added or deleted in a region other than the C-terminal or N-terminal of the amino acid sequence as set forth in any one of SEQ ID NOs: 28 and 30, provided that the altered amino acid sequences substantially retain the function similar to that of the heavy chain variable region of the antibody.

In some embodiments of the second aspect, the C-terminal or N-terminal region of the amino acid sequence as set forth in any one of SEQ ID NOs: 29 and 31 can also be truncated by about 1, 2, 3, 4, 5, 6, 7, 8, 9, 10, 11, 12, 13, 14, 15, 20, 25, or more amino acids, while the resulting amino acid sequences still retain the function similar to that of the light chain variable region of the antibody.

In some embodiments of the second aspect, 1, 2, 3, 4, 5, 6, 7, 8, 9, 10, 11, 12, 13, 14, 15, 20, 25, or more amino acids can also be added to the C-terminal or N-terminal region of the amino acid sequence as set forth in any one of SEQ ID NOs: 29 and 31, while the resulting amino acid sequences still retain the function similar to that of the light chain variable region of the antibody.

In some embodiments of the second aspect, 1, 2, 3, 4, 5, 6, 7, 8, 9, 10, 11, 12, 13, 14, 15, 20, 25, or more amino acids can also be added or deleted in a region other than the C-terminal or N-terminal of the amino acid sequence as set forth in any one of SEQ ID NOs: 29 and 31, provided that the altered amino acid sequences substantially retain the function similar to that of the light chain variable region of the antibody.

In some embodiments of any one of the first and second aspects, the antibody is a neutralizing antibody.

In some embodiments of any one of the first and second aspects, the antibody can be capable of binding to F protein of human respiratory syncytial virus (RSV).

In some specific embodiments of any one of the first and second aspects, F protein of the human respiratory syncytial virus (RSV) is a recombinant F protein of the human RSV, such as the recombinant F protein of the human RSV as set forth in SEQ ID NO: 12 or 13.

In some embodiments of any one of the first and second aspects, the antibody is a Fab fragment, a complete antibody, a F (ab')₂ fragment, or a single chain Fv fragment (scFv).

In some specific embodiments of any one of the first and second aspects, the antibody is a Fab fragment.

In some embodiments of any one of the first and second aspects, the antibody is a monoclonal antibody.

In some embodiments of any one of the first and second aspects, the antibody comprises a heavy chain constant region of an IgG1 subtype, an IgG2 subtype, or an IgG4 subtype.

In some embodiments of any one of the first and second aspects, the heavy chain constant region comprises a sequence of the Fc fragment of the heavy chain constant region of the IgG1 subtype, and the amino acids at positions 252, 254, 256 of the sequence of the Fc fragment are Y, T, and E, respectively, wherein the amino acid positions of the constant region of the antibody is determined according to EU numbering.

In some embodiments of any one of the first and second aspects, the antibody comprises a light chain constant region of a kappa subtype or a lambda subtype.

In a third aspect, there is provided in the present application a nucleic acid molecule encoding the antibody of the first or second aspect.

In some embodiments of the third aspect, the nucleic acid molecule is operably linked to a regulatory amino acid sequence that can be recognized by a host cell transformed with the vector.

In a fourth aspect, there is provided in the present application a pharmaceutical composition comprising the antibody of the first or second aspect and a pharmaceutically acceptable excipient, diluent or carrier.

In some embodiments of the fourth aspect, the pharmaceutical composition is for the prevention or treatment of an RSV-associated disease.

In some embodiments of the fourth aspect, the RSV-associated disease is a respiratory tract infection such as bronchiolitis and pneumonia.

In some embodiments of the fourth aspect, the pharmaceutical composition can further comprise one or more of a lubricant, such as talc, magnesium stearate, and mineral oil; a wetting agent; an emulsifier; a suspending agent; a preservative such as benzoic acid, sorbic acid and calcium propionate; a sweetening agent and/or a flavoring agent and the like.

In some embodiments of the fourth aspect, the pharmaceutical compositions of the present application can be formulated as a tablet, a pill, a powder, a lozenge, an elixir, a suspension, an emulsion, a solution, a syrup, a suppository, or a capsule, and the like.

In some embodiments of the fourth aspect, the pharmaceutical compositions of the present application can be delivered using any physiologically acceptable administration route including, but not limited to, oral administration, parenteral administration, nasal administration, rectal administration, intraperitoneal administration, intravascular injection, subcutaneous administration, transdermal administration, inhalation administration, and the like.

In some embodiments of the fourth aspect, the pharmaceutical composition for therapeutic use can be formulated for storage in the form of a lyophilized formulation or an aqueous solution by mixing a reagent with desired purity with a pharmaceutically acceptable carrier, excipient, etc. as appropriate.

In a fifth aspect, there is provided in the present application use of the antibody of the first or second aspect, the nucleic acid molecule of the third aspect, or the pharmaceutical composition of the fourth aspect in the manufacture of a medicament for the prevention or treatment of an RSV-associated diseases.

In some embodiments of the fifth aspect, the RSV-associated disease is a respiratory tract infection, such as bronchiolitis and pneumonia.

In a sixth aspect, there is provided in the present application provides a method of preventing or treating an RSV-associated disease, comprising administering to a subject in need thereof the antibody of the first or second aspect, or the pharmaceutical composition of the fourth aspect.

In some embodiments of the sixth aspect, the RSV-associated disease is a respiratory tract infection, such as bronchiolitis and pneumonia.

In a seventh aspect, there is provided in the present application a plasmid combination for expressing antibody Fab fragments comprising a first plasmid and a second plasmid, wherein
the first plasmid comprises a heavy chain VH-CH1 expression unit of the antibody and a first recombination site;
the second plasmid comprises a light chain VL-CL expression unit of the antibody and a second recombination site; and
the first recombination site is different from the second recombination site.

In some embodiments of the seventh aspect, the plasmid combination comprises a plurality of the first plasmids, and the plurality of the first plasmids carry different heavy chain VH-CH1 expression units of the antibody and have the same first recombination site.

In some embodiments of the seventh aspect, the plasmid combination comprises a plurality of the second plasmids, and the plurality of the second plasmids carry different light chain VL-CL expression units of the antibody and have the same second recombination site.

In an eighth aspect, there is provided in the present application a recombinant system for expressing a library of antibody Fab fragments comprising a first plasmid, a second plasmid, and a cell expressing a phage integrase; wherein
the first plasmid comprises a heavy chain VH-CH1 expression unit of the antibody and a first recombination site;
the second plasmid comprises a light chain VL-CL expression unit of the antibody and a second recombination site; and
the first recombination site is different from the second recombination site.

In some embodiments of the eighth aspect, the recombinant system comprises a plurality of the first plasmids, and the plurality of the first plasmids carry different heavy chain VH-CH1 expression units of the antibody and have the same first recombination site.

In some embodiments of the eighth aspect, the recombinant system comprises a plurality of the second plasmids, and the plurality of the second plasmids carry different light chain VL-CL expression units of the antibody and have the same second recombination site.

In some embodiments of the eighth aspect, wherein the first plasmid, the second plasmid, and the cell expressing the phage integrase are each exist independently, or at least one of the first plasmid and the second plasmid has been introduced into the cell expressing the phage integrase.

In some specific embodiments of the eighth aspect, the second plasmid has been introduced into the cell expressing the phage integrase.

In a ninth aspect, there is provided in the present application a recombinant cell for expressing a library of antibody Fab fragments comprising a first plasmid, a second plasmid and expressing a phage integrase; wherein
the first plasmid comprises a heavy chain VH-CH1 expression unit of the antibody and a first recombination site;
the second plasmid comprises a light chain VL-CL expression unit of the antibody and a second recombination site; and
the first recombination site is different from the second recombination site.

In some embodiments of the ninth aspect, the recombinant cell comprises a plurality of the first plasmids, and the plurality of the first plasmids carry different heavy chain VH-CH1 expression units of the antibody and have the same first recombination site.

In some embodiments of the ninth aspect, the recombinant cell comprises a plurality of the second plasmids, and the plurality of the second plasmids carry different light chain VL-CL expression units of the antibody and have the same second recombination site.

In some embodiments of the ninth aspect, the recombinant cell is a prokaryotic cell.

In some embodiments of the ninth aspect, the recombinant cell is *E*. *coli.*

In some embodiments of the ninth aspect, the recombinant cell is a genetically engineered bacterium.

In some specific embodiments of the ninth aspect, the recombinant cell is a male *E. coli* strain having factor F, such as a TG1 strain.

In a tenth aspect, there is provided in the present application a method of preparing a library of antibody Fab fragments, comprising transducing a first plasmid and a second plasmid into a cell expressing a phage integrase; wherein
the first plasmid comprises a heavy chain VH-CH1 expression unit of the antibody and a first recombination site;
the second plasmid comprises a light chain VL-CL expression unit of the antibody and a second recombination site; and
the first recombination site is different from the second recombination site.

In some embodiments of the tenth aspect, a plurality of the first plasmids are transduced into the cell expressing the phage integrase, wherein the plurality of the first plasmids carry different heavy chain VH-CH1 expression units of the antibody and have the same first recombination site.

In some embodiments of the tenth aspect, a plurality of the second plasmids are transduced into the cell expressing the phage integrase, wherein the plurality of the second plasmids carry different light chain VL-CL expression units of the antibody and have the same second recombination site.

In some embodiments of the tenth aspect, transducing the first plasmid and/or the second plasmid into the cell expressing the phage integrase comprises the steps of:
(1) transducing the first plasmid and/or the second plasmid into a competent cell;
(2) infecting the competent cell obtained in step (1) with a helper phage to construct a phage library; and
(3) transducing the phage library obtained in step (2) into the cell expressing the phage integrase.

In some embodiments of the tenth aspect, the competent cell is a prokaryotic cell.

In some embodiments of the tenth aspect, the competent cell is *E. coli.*

In some embodiments of the tenth aspect, the competent cell is a genetically engineered bacterium.

In some specific embodiments of the tenth aspect, the competent cell is a male *E*. *coli* strain having factor F, such as a TG1 strain.

In some embodiments of the tenth aspect, the competent cell does not express a phage integrase.

In some embodiments of the tenth aspect, the helper phage is an M13 helper phage.

In some embodiments of any one of the seventh to tenth aspects, the first plasmid is a phagemid.

In some embodiments of any one of the seventh to tenth aspects, the second plasmid is a phagemid.

In some embodiments of any one of the seventh to tenth aspects, the first plasmid and the second plasmid comprise different replication origins.

In some embodiments of any one of the seventh to tenth aspects, the replication origin is selected from the group consisting of one or more of a pBR ori, a CDF ori, a replication origin of M13 filamentous phage (f1 ori) and a p15A ori.

In some specific embodiments of any one of the seventh to tenth aspects, the first plasmid comprises a pBR ori and an f1 ori.

In some specific embodiments of any one of the seventh to tenth aspects, the second plasmid comprises a CDF ori.

In some embodiments of any one of the seventh to tenth aspects, the first recombination site and the second recombination site are sites for phage attachment in each genome of the phage and its host bacteria, respectively.

In some embodiments of any one of the seventh to tenth aspects, the first recombination site is attP.

In some embodiments of any one of the seventh to tenth aspects, the second recombination site is attB.

In some specific embodiments of any one of the seventh to tenth aspects, the first recombination site comprises a nucleotide sequence as set forth in SEQ ID NO:2 or a nucleotide sequence obtained from a nucleotide sequence as set forth in SEQ ID NO:2 by one or more substitutions, deletions, or additions that do not essentially alter the function of the first recombination site.

In some specific embodiments of any one of the seventh to tenth aspects, the second recombination site comprises a nucleotide sequence as set forth in SEQ ID NO:4 or a nucleotide sequence obtained from a nucleotide sequence as set forth in SEQ ID NO:4 by one or more substitutions, deletions, or additions that do not essentially alter the function of the second recombination site.

In some embodiments of any one of the seventh to tenth aspects, the number of the one or more substitutions, deletions or additions that do not essentially alter the function of the first recombination site or the second recombination site is 1 to 30, preferably 1 to 20, more preferably 1 to 10, wherein the resulting nucleotide sequence substantially retains unaltered function relative to that of the first recombination site or the second recombination site.

In some embodiments of any one of the seventh to tenth aspects, the first plasmid and the second plasmid are different.

In some specific embodiments of any one of the seventh to tenth aspects, the first plasmid is a phagemid and the second plasmid is a prokaryotic expression vector *(e.g.,* a bacterial plasmid).

In some specific embodiments of any one of the seventh to tenth aspects, the first plasmid is represented as pHGDisn-attP-new.

In some specific embodiments of any one of the seventh to tenth aspects, the second plasmid is epresented as pHKb-attB-new.

In some embodiments of any one of the seventh to tenth aspects, the first recombination site is located at a polyclonal enzyme cleavage site of the first plasmid.

In some embodiments of any one of the seventh to tenth aspects, the second recombination site is located at a polyclonal enzyme cleavage site of the second plasmid.

In some specific embodiments of any one of the seventh to tenth aspects, the first recombination site is located between the XbaI and KpnI enzyme cleavage sites of the pHGDisn-attP-new plasmid.

In some specific embodiments of any one of the seventh to tenth aspects, the second recombination site is located between the HindIII and BamHI enzyme cleavage sites of the pHKb-attB-new plasmid.

In some embodiments of any one of the seventh to tenth aspects, the light chain constant region CL of the antibody is a human kappa light chain constant region or a human lambda light chain constant region.

In some embodiments of any one of the seventh to tenth aspects, the heavy chain constant region CH1 fragment of the antibody is IgG1, IgG2, IgG3, or IgG4 subtype.

In some embodiments of any one of the seventh to tenth aspects, the first plasmid and/or the second plasmid comprises a resistance gene coding region.

In some embodiments of any one of the seventh to tenth aspects, the 3' end of the nucleic acid molecule comprising the nucleic acid molecule encoding the CH1 fragment of the heavy chain constant region of the antibody is fused to the 5' end of the nucleic acid molecule encoding protein gIII of the filamentous phage M13.

In some embodiments of any one of the seventh to tenth aspects, the presence of the resistance gene facilitates screening of the recombinant system or the recombinant cell.

In some embodiments of any one of the seventh to tenth aspects, the resistance gene is an antibiotic resistance gene.

In some embodiments of any one of the seventh to tenth aspects, the resistance gene is selected from the group consisting of a chloramphenicol resistance gene (CmR), an ampicillin resistance gene (Ampr), a kanamycin resistance gene (KaR), and a tetracycline resistance gene (TetR).

In some embodiments of any one of the eighth to tenth aspects, the phage integrase is a tyrosine integrase.

In some embodiments of any one of the eighth to tenth aspects, the tyrosine integrase is a lambda phage integrase.

In some embodiments of any one of the eighth to tenth aspects, the phage integrase is an inducibly expressed or constitutively expressed integrase.

In some embodiments of any one of the eighth to tenth aspects, the phage integrase is an inducibly expressed integrase.

In some embodiments of any one of the eighth to tenth aspects, the inducible expression is carried out by utilizing an inducible promoter, such as a lactose promoter (Plac) or an arabinose promoter (Para).

In some embodiments of the eighth or tenth aspect, the cell expressing the phage integrase is a prokaryotic cell.

In some embodiments of the eighth or tenth aspect, the cell expressing the phage integrase is *E. coli.*

In some embodiments of the eighth or tenth aspect, the cell expressing the phage integrase is a genetically engineered bacterium.

In some embodiments of the eighth aspect or the tenth aspect, the cell expressing a phage integrase is a male *E. coli* strain with factor F.

In some specific embodiments of the eighth or tenth aspect, the cell expressing the phage integrase is a TG1 strain.

In some specific embodiments of any one of the seventh to tenth aspects, the first plasmid comprises the heavy chain VH-CH1 expression unit of the antibody and an attP attachment site; and the second plasmid comprises the light chain VL-CL expression unit of the antibody and an attB attachment site.

In some embodiments of the seventh aspect, the first recombination site and the second recombination site correspond to each other. For example, when the nucleotide sequence of the first recombination site is set forth in SEQ ID NO:2, the nucleotide sequence of the second recombination site is set forth in SEQ ID NO:4.

In some embodiments of the eighth or tenth aspect, there is a correspondence among the first recombination site, the second recombination site, the type of phage integrase, and the type of the cell expressing phage integrase.

In some embodiments of the eighth or tenth aspect, the second recombination site, the type of phage integrase, and the type of the cell expressing phage integrase applicable to the present application can be determined once the first recombination site is determined.

In some embodiments of the eighth or tenth aspect, the first recombination site, the type of phage integrase, and the type of the cell expressing phage integrase applicable to the present application can be determined once the second recombination site is determined.

In some embodiments of the eighth or tenth aspect, when the phage integrase type is determined, the type of the cell expressing phage integrase applicable to the present application can be determined once the type of phage integrase is determined.

In some embodiments of the eighth or tenth aspect, when the nucleotide sequence of the first recombination site is set forth in SEQ ID NO:2, the nucleotide sequence of the second recombination site is set forth in SEQ ID NO:4, the phage integrase is lambda phage integrase, and the cell expressing the phage integrase is TG1 *E. coli.*

In some embodiments of the ninth aspect, there is a correspondence among the first recombination site, the second recombination site, the type of phage integrase, and the type of recombinant cell.

In some embodiments of the ninth aspect, the second recombination site, the type of phage integrase, and the type of recombinant cell applicable to the present application can be determined once the first recombination site is determined.

In some embodiments of the ninth aspect, the first recombination site, the type of phage integrase, and the type of recombinant cell applicable to the present application can be determined once the second recombination site is determined.

In some embodiments of the ninth aspect, when the type of recombinant cell applicable to the present application can be determined once the type of phage integrase is determined.

In some embodiments of the ninth aspect, when the nucleotide sequence of the first recombination site is set forth in SEQ ID NO:2, the nucleotide sequence of the second recombination site is set forth SEQ ID NO:4, the phage integrase is lambda phage integrase, and the recombinant cell is TG1 *E. coli.*

In some specific embodiments of the tenth aspect, the method of preparing the library of antibody Fab fragments can comprise the steps of:
(1) transducing the first plasmid *(e.g.,* a plurality of first plasmids such as a plurality of phagemids carrying different heavy chain VH-CH1 expression units of the antibodies and the same attP attachment site) into the competent cells *(e.g.,* TG1 competent cells);
(2) infecting the competent cells obtained in step (1) with a helper phage *(e.g.,* M13 helper phage) to construct a phage library *(e.g.,* a phage library comprising a plurality of phages carrying different heavy chain VH-CH1 expression units of the antibodies and the same attP attachment site);
(3) transducing the second plasmid (*e.g.,* a plurality of second plasmids carrying different light chain VL-CL expression units of the antibodes and the same attB attachment site) into the cells expressing phage integrase (*e*.*g*., the TG1 competent cells expressing lambda phage integrase);
(4) inducing the cells obtained in step (3) to express a phage integrase (*e*.*g*., lambda phage integrase);
(5) infecting the cells expressing phage integrase obtained in step (4) with the phage library (*e.g.,* a phage library comprising a plurality of phages carrying different heavy chain VH-CH1 expression units of the antibodies and the same attP attachment site); and
(6) infecting the cells obtained in step (5) with a helper phage (*e.g.,* M13 helper phage) to construct the library of antibody Fab fragments.

In an eleventh aspect, there is provided in the present application use of the plasmid combination of the seventh aspect, the recombinant system of the eighth aspect, or the recombinant cell of the ninth aspect in the manufacture of a library of antibody Fab fragments.

In other aspects, there is also provided in the present application a vector comprising a nucleic acid molecule encoding the antibody or the light or heavy chain thereof of the invention, a host cell comprising the vector, and a method of producing the antibody. In some embodiments, the nucleic acid molecule is operably linked to a control sequence that can be recognized by the host cells transformed with the vector. In some embodiments, the method of producing the antibody comprises culturing the host cells. In some embodiments, the method of producing the antibody further comprises recovering the antibody from the host cell culture medium.

In addition, the antibody specific for RSV described herein can also be used to detect the presence of RSV in a biological sample. Antibody-based detection methods are well known in the art and include, for example, ELISA, immunoblot, radioimmunoassay, immunofluorescence, immunoprecipitation, and other related techniques.

It should be understood that the foregoing detailed description is only intended to enable those skilled in the art to have better understanding of the present application and is not intended to be limiting in any aspect. Various modifications and variations can be made to the described embodiments by those skilled in the art.

### Examples

The following examples are for purposes of illustration only and are not intended to limit the scope of the present application.

### Example 1: Preparation of a library of fully human recombinant Fabs

The example was implemented with reference to Chinese Invention Patent No. CN 108251431B, which is incorporated herein by reference.

### 1.1 Construction of recombinant expression plasmids containing attB and attP attachment sites

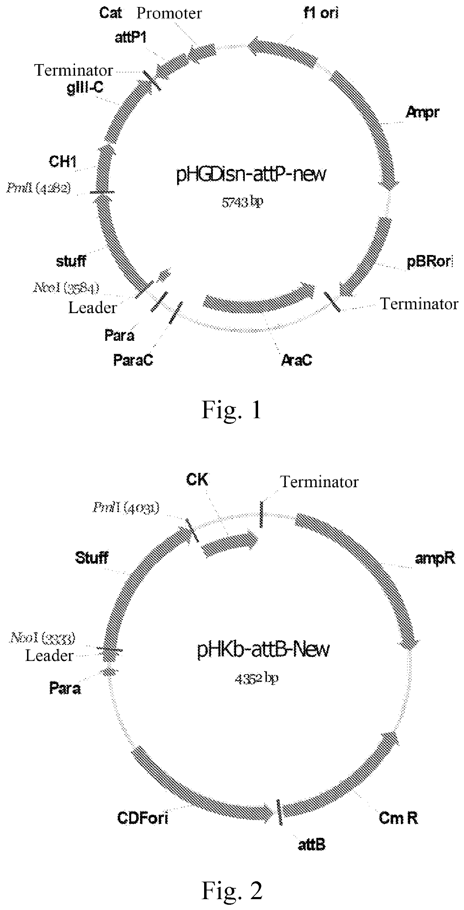

Based on the pADG-S plasmid, the phagemid vector pHGDisn-attP-new containing attP attachment site and expressing the heavy chain Fd (VH-CH1) domain of the antibody was constructed by cloning a synthetic recombinant gene fragment containing a chloramphenicol resistance gene promoter (Cat promoter, with the coding sequence as set forth in SEQ ID NO: 1) and an attP attachment site (attP1, with the coding sequence as set forth in SEQ ID NO: 2) into the vector pADG-S using double enzyme digestion with XbaI and KpnI(Figure 1).

Based on the pADK-S plasmid, the prokaryotic expression vector pHKb-attB-new containing an attB attachment site and expressing a light chain of the antibody was constructed by cloning a synthetic recombinant gene fragment containing a CDF replication origin (CDF ori, with the coding sequence as set forth in SEQ ID NO: 3), an attB attachment site (attB1, with the coding sequence as set forth in SEQ ID NO: 4), a chloramphenicol resistance gene (CmR, with the coding sequence as set forth in SEQ ID NO: 5) and a complete ampicillin resistance gene expression element (AmpR, with the coding sequence a set forth in SEQ ID NO: 6) into the vector pADK-S using double enzyme digestion with HindIII and BamHI (Figure 2).

PHGDisn-attP-new and pHKb-attB-new comprise different replication origins and can replicate and coexist within the same *E. coli* cell. After the lambda phage integrase (λInt) induces specific recombination of the attP and attB attachment sites, the new formed plasmid can express chloramphenicol acetyl transferase and exhibit chloramphenicol resistance because it contains a complete chloramphenicol promoter and a coding gene, thereby facilitating the screening of recombinant plasmids.

### 1.2 Preparation of λInt gene-edited TG1 E. coli

The attL site and the attR site were generated by the specific recombination of the attB and attP attachment sites in *E*. *coli* cells, and the reaction must be carried out under the catalysis of lambda phage integrase¹⁴. The gene encoding the lactose operon-inducible lambda phage integrase expression element (with the nucleotide sequence as set forth in SEQ ID NO: 7) was inserted into the genome of TG1 *E. coli* by gene editing means. TG1-λInt recombinant engineering bacteria were prepared by GenScript Biotech Corporation.

### 1.3 Construction of an antibody library of fully human heavy chains

With reference to Chinese Invention Patent No. CN 108251431B, an antibody library of fully human heavy chains with the diversity of 1.73E+9 was constructed by employing the frozen cDNA of the natural human heavy chain variable region and the PCR products of VH1, VH3 and VH5 with mutations as templates, designing PCR primers to amplify fragments of interest, cloning the fragments of interest into the vector pHGDisn-attP-new using double enzyme digestion with NcoI and PmlI, and electrotransforming the ligation products into TG1 competent cells. The constructed antibody library was sequenced and analysized, with an average accuracy of over 85%.

### 1.4 Construction of an antibody library of fully human light chains

With reference to Chinese Invention Patent No. CN 108251431B, an antibody library of fully human light chain with the diversity of 1.95E+8 was constructed by employing the frozen cDNA of the natural human light chain variable region and the PCR products of VK1 and VK3 with mutations as templates, designing PCR primers to amplify fragments of interest, cloning the fragments of interest into the vector pHKb-attB-new using double enzyme digestion with NcoI and PmlI, and electrotransforming the ligation products into TG1-λInt competent cells. The constructed antibody library was sequenced and analysized, with an average accuracy of over 90%.

### 1.5 Preparation of a library of fully human recombinant Fabs

The bacteria solution containing the heavy chain library constructed as described above was inoculated into 200mL liquid medium, cultured to a logarithmic growth phase at 37°C and 220rpm, infected with M13 helper phage, and cultured overnight at 28°C and 220rpm to amplify the phage. Then, the purified heavy chain phage library was prepared using PEG/NaCl precipitation method. The purified heavy chain phage library was stored in a - 80°C refrigerator for spare use after its titer was measured.

The antibody library of fully human light chains prepared as described above was inoculated into 50mL liquid medium with IPTG at a final concentration of 0.1mM, cultured to a logarithmic growth phase at 37°C and 220rpm, and then infected with the heavy chain phage library. After infection, the bacterial liquid was evenly spread over a bacterial culture dish, and incubated overnight in a constant temperature incubator at 32°C.

The bacteria grown on the dish were counted and sent for sequencing to identify the recombinant sites of the monoclones as well as the light and heavy chain sequences. The post-recombinant library capacity was counted by the dilution method. The total number of recombinant colonies was calculated as 3.80E+12. The results of monoclonal sequencing showed that all of the clones grown on the dish were recombinant plasmids containing the newly generated attL (attL1, with the coding sequence as set forth in SEQ ID NO: 8) and attR (attR1, with the coding sequence as set forth in SEQ ID NO: 9) recombination sites, the complete light chain genes and the heavy chain Fd gene, with more than 76.5% correct antibody genes.

Colonies on the above bacterial culture dish were collected, inoculated into a liquid medium, and cultured at 37°C and 220rpm until a logarithmic growth phase, and then infected with M13 helper phage to prepare a purified human Fab phage library.

### Example 2: Preparation of F protein of RSV and the recombinant antibodies

### 2.1 Preparation of F protein of RSV

Recombinant F protein of different subtypes of viruses, including F protein of strain A2 of RSV subtype A (F-A2, with the amino acid sequence as set forth in SEQ ID NO: 10) and F protein of strain 18537 of RSV subtype B (F-18537, with the amino acid sequence as set forth in SEQ ID NO: 11), was required in the preparation of monoclonal antibodies against F protein of RSV.Based on two different subtypes of F protein of RSV, the inventors constructed F protein mutants of DS-Cav1 structure¹⁵ before fusion, designated as RSV-DS-Cav1-A (with the amino acid sequence as set forth in SEQ ID NO: 12) and RSV-DS-Cav1-B (with the amino acid sequence as set forth in SEQ ID NO: 13), respectively. There are post-translational modifications (e.g., glycosylation or disulfide bonds, etc) in F protein. Therefore, the use of a mammal cell expression system would be more favorable for maintaining the structure and function of the recombinant protein. Meanwhile, the addition of His tag (His, with amino acid sequence as set forth in SEQ ID NO:14) to the C-terminus of the recombinant protein will be more favorable for the purification of the recombinant protein and the identification of the function of the monoclonal antibodies.

The RSV-DS-Cav1-A and RSV-DS-Cav1-B genes were synthesized. The synthesized genes were cloned into a suitable eukaryotic expression vector (*e.g*., pcDNA3.1 from Invitrogen Inc., etc.) using conventional molecular biology techniques. HEK293 cells (*e.g.,* HEK293F from Invitrogen Inc., etc.) were transfected with the prepared recombinant protein expression plasmids using liposomes (*e.g*., 293fectin from Invitrogen Inc., etc.) or other cationic transfection reagent (*e.g.,* PEI, etc.). The cells were cultured in suspension in serum-free medium for 3-4 days. The culture supernatant was then harvested by centrifugation and other means. The recombinant protein in the supernatant was subjected to one-step purification using a metal chelate affinity chromatography column (e.g., HisTrap FF from GE Inc., etc.). The recombinant protein preservation buffer was then replaced with PBS (pH 7.0) or other suitable buffers using a desalting column ((e.g., Hitrap desaulting from GE Inc., etc.). If necessary, the sample can be sterilized by filtration and then stored in aliquots at - 20°C.

### 2.2 Preparation of the recombinant antibodies

The nucleotide sequences encoding the heavy chain variable region and the light chain variable region of the antibodies were cloned into eukaryotic expression vectors (e.g., pcDNA3.1 from Invitrogen Inc., etc.) fused with the nucleotide sequences encoding the heavy chain constant region and the light chain constant region, respectively, by conventional molecular biological means. The complete antibodies were expressed in combination. The heavy chain constant region of the antibodies can be a human IgG1 subtype (with the amino acid sequence as set forth in SEQ ID NO: 15), a human IgG1 subtype mutant IgG1-YTE (with the amino acid sequence as set forth in SEQ ID NO: 16), a murine IgG2a subtype (with the amino acid sequence as set forth in SEQ ID NO: 17), and the light chain constant region of the antibodies can be a human kappa subtype (with the amino acid sequence of as set forth in SEQ ID NO: 18), a human lambda subtype (with the amino acid sequence as set forth in SEQ ID NO: 19), a murine kappa subtype (with the amino acid sequence as set forth in SEQ ID NO: 20) or a murine lambda subtype (with the amino acid sequence as set forth in SEQ ID NO: 21).

HEK293 cells (*e.g*., HEK293F from Invitrogen Inc.) were transfected with the prepared recombinant antibody expression plasmids using liposomes (*e.g*., 293fectin from Invitrogen, Inc., etc.) or other transfection reagents (*e.g.,* PEI, etc.). The cells were cultured in suspension in serum-free medium for 3-5 days. The culture supernatant was then harvested by centrifugation and other means. The recombinant antibodies in the supernatant was subjected to one-step purification using Protein A/G affinity chromatography column (*e.g.,* Mabselect SURE from GE Inc, etc.). The recombinant protein preservation buffer was then replaced with PBS (pH 7.0) or other suitable buffers using a desalting column (*e.g*., Hitrap desaulting from GE Inc., etc.). If necessary, the sample can be sterilized by filtration and then stored in aliquots at -20°C.

### Example 3: Screening of a library of fully human recombinant Fabs with F protein of RSV

Referring to the reference (see the Chinese Patent Application No. 201510097117.0 for detailed experimental protocols, which is incorporated by reference in its entirety), the library of fully human recombinant Fab constructed in Example 1 was screened by a solid phase screening strategy (as for the experimental protocol, see, Phage Display: A Practical Approach, Clackson, T. (USA) and Lowman, H. B. (USA) (ed.), translated by Lan Ma et al., Chemical Industry Press, May 2008) using the recombinant F protein of RSV prepared in Example 2. Four to six rounds of screening were carried out by means of binding, elution, neutralization, infection and amplification. Finally, two Fab antibodies R3B1h1 and R22B1 specifically binding to F protein of RSV were obtained.

### Example 4: Identification of the recombinant monoclonal antibodies against F protein of RSV

The nucleotide sequences encoding the light and heavy chains of two molecules R3B1h1 and R22B1 were cloned into eukaryotic expression vectors using conventional molecular biological methods, respectively, so as to prepare a recombinant human IgG1-κ version of the monoclonal antibodies. Meanwhile, the human anti-RSV monoclonal antibody Palivizumab (Hu1129) (with the amino acid sequence of the heavy chain variable region as set forth in SEQ ID NO: 22 and the amino acid sequence of the light chain variable region as set forth in SEQ ID NO: 23) was prepared with reference to the patent No. US 7704505 B2, the human anti-RSV monoclonal antibody Nirsevimab (MEDI8897) (with the amino acid sequence of the heavy chain variable region as set forth in SEQ ID NO: 24 and the amino acid sequence of the light chain variable region as set forth in SEQ ID NO: 25) was prepared with reference to the patent No. US 11186628 B2, and the human anti-RSV monoclonal antibody Clesrovimab (RB1) (with the amino acid sequence of the heavy chain variable region as set forth in SEQ ID NO: 26 and the amino acid sequence of the light chain variable region as set forth in SEQ ID NO: 27) was prepared with reference to the patent No. US 9963500 B2, the three antibodies were used as positive control antibodies.

### 4.1 Verification of the binding activity of recombinant monoclonal antibodies against F protein of RSV

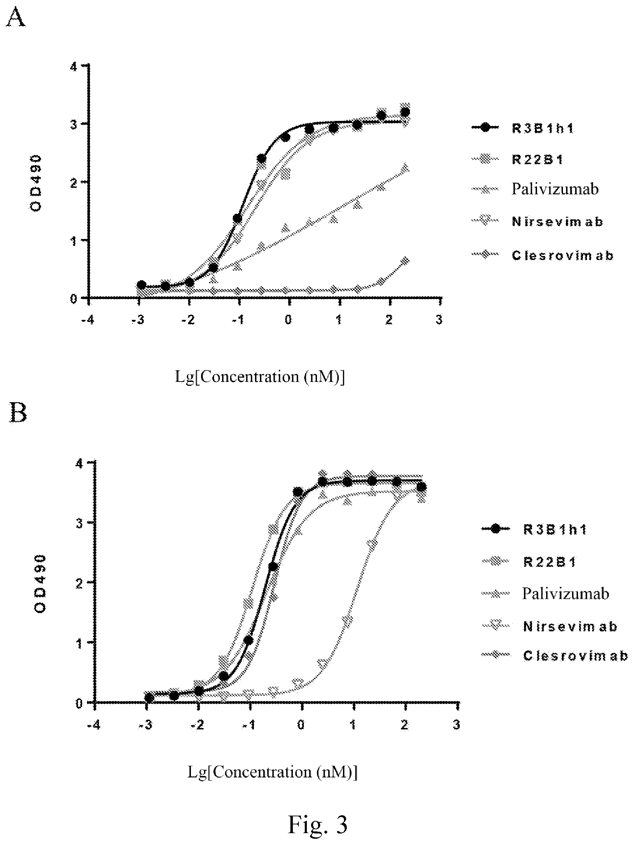

96-well ELISA plates were coated with the prepared RSV-DS-Cav1-A and RSV-DS-Cav1-Bat 3µg/mL, 100µL/well, and at 4°C overnight, respectively. After the plates were blocked using a blocking solution (PBS-0.1% Tween 20-3% milk (PBST-3% milk)) at 37°C for 1 hour, each recombinant monoclonal antibody against F protein of RSV was added to the corresponding plate, respectively, and bound at 37°C for 1 hour. The ELISA plates were washed with PBS-0.1% Tween 20 (PBST) buffer and HRP-mouse anti-human IgG (Bioss, bsm-0297M-HRP) was added and bound at 37°C for 1 hour. The ELISA plates were washed with PBST buffer. The OPD substrate colour developing solution was added. After incubation for 5-10 minutes, the colour development was terminated with 1M H₂SO₄. The optical density values were measured at dual wavelengths of 492nm/630nm using a microplate reader. The results of ELISA analysis (Figure 3) showed that the recombinant monoclonal antibodies R3B1h1 and R22B1 against F protein of RSV have comparable activities in binding the two recombinant proteins RSV-DS-Cav1-A and RSV-DS-Cav1-B.

### 4.2 Epitope analysis of the recombinant monoclonal antibodies against F protein of RSV Virus

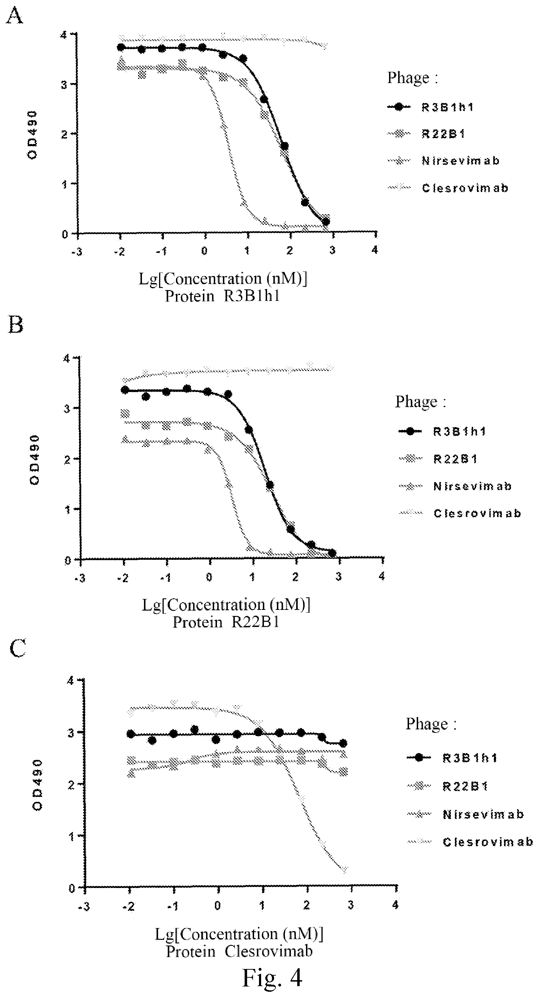

96-well ELISA plates were coated with the recombinant protein RSV-DS-Cav1-B (1µg/mL, 100µL/well) overnight in a refrigerator at 4°C. The plates were blocked with a blocking solution (PBST-3% milk) at 37°C for 1 hour. Gradient dilutions of the recombinant monoclonal antibodies against F protein of RSV (R3B1h1/R22B1/Clesrovimab) were performed with each of the purified phages against F protein of RSV (R3B1h1/R22B1/Clesrovimab/Nirsevimab) at a fixed concentration (1×10¹¹cfu/mL), respectively. The starting concentration of the antibodies was 200µg/mL, and the dilution was carried out at 3-fold for a total of 10 concentration gradients. The antibody dilutions were added to the blocked 96-well ELISA plates at 100 µL/well and the plates were incubated at 37°C for 1 hour. The ELISA plates were washed with PBST buffer, followed by the addition of HRP anti-M13 secondary antibody (Sino Biological Inc., 11973-MM05T-H) and the plates were incubated at 37°C for 1 hour. The ELISA plates were washed with PBST, OPD substrate colour developing solution was added. After incubation for 5-10 minutes, the colour development was terminated with 1M H₂SO₄. The optical density values were measured at dual wavelengths of 492nm/630nm using a microplate reader. The results of ELISA analysis were showed in Figure 4. The monoclonal antibody R3B1h1 can block the binding of the phages R22B1/Nirsevimab to the recombinant protein RSV-DS-Cav1-B, and have no effect on the binding of the phage Clesrovimab to the recombinant protein RSV-DS-Cav1-B (Figure 4A). The monoclonal antibody R22B1 can block the binding of the phages R3B1h1/Nirsevimab to the recombinant protein RSV-DS-Cav1-B, and have no effect on the binding of the phage Clesrovimab to the recombinant protein RSV-DS-Cav1-B (Figure 4B). The monoclonal antibody Clesrovimab cannot block the binding of the phages R3B1h1/R22B1/Nirsevimab to the recombinant protein RSV-DS-Cav1-B (Figure 4C).

### 4.3 Affinity analysis of the recombinant monoclonal antibodies against F protein of RSV

The affinity of the monoclonal antibodies against F protein of RSV was determined by the surface plasmon resonance technique using Biacore T200. Related reagents and consumables such as an amino-coupling kit (BR-1000-50), a human antibody capture kit (BR-1008-39), a CM5 chip (BR100012), and 10×HBS-EP (pH 7.4, BR100669) were purchased from GE healthcare. The surface of the carboxylated CM5 chip was activated with 1-ethyl-3-(3-dimethylaminopropyl) carbodiimide hydrochlorid (EDC) and N-Hydroxysuccinimide (NHS) according to the instructions in the kit. An anti-human IgG (Fc) antibody (capture antibody) was diluted to 25µg/mL with 10mM sodium acetate (pH 5.0), and then injected at a flow rate of 10µL/min to achieve a coupling amount of approximately 10000 response units (RU). After injection of the capture antibody, 1M ethanolamine was injected to block the unreacted groups. For the kinetic measurements, the monoclonal antibodies against F protein of RSV were diluted to 0.5-1µg/mL, and injected at 10µL/min to ensure that approximately 80RU of the antibody was captured by the anti-human Fc antibody. RSV-DS-Cav1-A or RSV-DS-Cav1-B was set to a series of concentration gradients (e.g., 6.17nM, 18.5nM, 55.6nM, 166.7nM, 500nM), and injected at 25°C from lower to higher concentrations at 30µL/min, with an association time of 90s and a dissociation time of 3,600s. A 3M MgCl₂ solution was injected at 10µL/min for 30s to regenerate the surface of the chip. The association rate (Kₒₙ) and dissociation rate (K_{off}) were calculated by fitting the association and dissociation sensing grams with a 1:1 association model using Biacore T200 evaluation software version 3.0. The dissociation equilibrium constant (K_{D}) was calculated as the ratio K_{off}/Kₒₙ. The fitting results are shown in Tables 1 and 2.

**Table 1. Affinity constants for the binding of the recombinant monoclonal antibodies against F protein of RSV to RSV-DS-Cav1-A**

| | Kₒₙ (1/MS) | K_{off} (1/S) | K_{D} (M) |
|---|---|---|---|
| Palivizumab | 3.158E+4 | 8.197E-5 | 2.595E-9 |
| Clesrovimab | 2.001E+4 | 3.515E-4 | 1.756E-8 |
| Nirsevimab | 3.149E+4 | 1.502E-5 | 4.770E-10 |
| R3B1h1 | 3.184E+4 | 2.370E-5 | 6.213E-10 |
| R22B1 | 3.991E+4 | 1.411E-5 | 6.213E-10 |

**Table 2. Affinity constants for the binding of the recombinant monoclonal antibodies against F protein of RSV to RSV-DS-Cav1-B**

| | Kₒₙ (1/MS) | K_{off} (1/S) | K_{D} (M) |
|---|---|---|---|
| Palivizumab | 1.360E+5 | 2.001E-4 | 1.471E-9 |
| Clesrovimab | 9.171E+6 | 8.208E-5 | 8.951E-12 |
| Nirsevimab | 1.082E+5 | 3.327E-4 | 3.075E-9 |
| R3B1h1 | 2.286E+5 | 8.093E-5 | 3.541E-10 |
| R22B1 | 3.577E+5 | 1.010E-4 | 2.824E-10 |

### 4.4 Inhibition of the RSV infection of Hep-2 cells by the recombinant monoclonal antibodies against F protein of RSV

### 4.4.1 Inhibition of the RSV/A2 infection of Hep-2 cells by the recombinant monoclonal antibodies against F protein of RSV

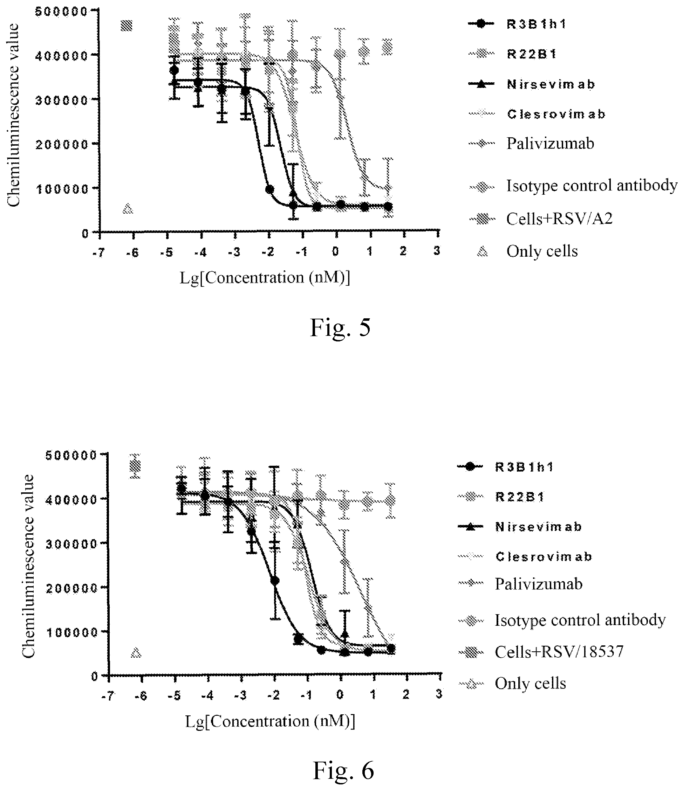

The monoclonal antibodies against F protein of RSV (R3B1h1, R22B1, Nirsevimab, Clesrovimab and Palivizumab) and negative isotype control antibody were diluted with Hep-2 cell maintenance medium (DMEM+2%FBS+1%P/S+2mM Glu) at a starting concentration of 66.7nM, with a 5-fold gradient for a total of 10 concentration gradients. The antibody dilutions were added to 96-well sterile fully permeable cell culture plates. 1 frozen virus solution RSV/A2 was placed in a P2 laboratory biosafety cabinet and slowly thawed, and diluted to 1.5×10³ PFU/mL with Hep-2 cell maintenance medium. The diluted virus solution was added to the diluted antibody at 70µL/well to be thoroughly mixed. At the same time, separate cell control wells (Hep-2 cell maintenance medium at 140µL/well) and cell+RSV/A2 wells (Hep-2 cell maintenance medium at 70µL/well +RSV/A2 dilution at 70µL/well) were set. After all samples were added, the plates were covered and placed in a cell culture chamber (at 37±1°C, 5±1% CO₂), and neutralized for 1 h. Hep-2 cells in a logarithmic growth phase were harvested, digested and centrifuged, and the counted cell suspension was diluted with Hep-2 cell maintenance medium to a single cell suspension of 4.3×10⁵ cells/mL. The cells were seeded into 96-well cell culture plates that had been neutralized at 70µL/well and incubated in a cell culture incubator (at 37±1°C, 5±1%CO₂) for 60-72h. After viral infiltration, the cells were washed twice with PBS, and fixed for 30 minutes in a refrigerator at 2~8°C with80% acetone. 80% acetone was then discarded. The cells were washed twice with PBS, and then the detection antibody Palivizumab (100µL/well) diluted to 10µg/mL with PBS was added and incubated at 37°C in the dark for 60 min. After the primary antibody was discarded, the cells were washed twice with PBS. Then the FITC-labeled goat anti-human IgG (ZSGB-BIO, ZF-0308) diluted with PBS (1: 400), was added at 100µL/well, and incubated at 37°C in the dark for 40 min. After the secondary antibody was discarded, the cells were washed twice with PBS. The chemiluminescence values were detected by Rapid Fluorescent Focus Inhibition Test (RFFIT) using a multifunctional microplate reader (SpectraMax I3). The results showed (Figure 5) that R3B1h1 has the best activity, which is about 5-fold higher than that of Nirsevimab, about 12-fold higher than that of Clesrovimab, and about 410-fold higher than that of Palivizumab, based on the comparison of the IC₅₀ values (Table 3).

**Table 3. Semi-inhibitory concentrations of the monoclonal antibodies against F protein of RSV inhibiting RSV/A2 strain infection of Hep-2 cells**

| Name of Antibody | IC₅₀(nM) |
|---|---|
| R3B1h1 | 0.005162 |
| R22B1 | 0.07067 |
| Nirsevimab | 0.02306 |
| Clesrovimab | 0.06219 |
| Palivizumab | 2.12 |
| Isotype control antibody | / |

### 4.4.2 Inhibition of the RSV/18537 infection of Hep-2 cells by the recombinant monoclonal antibodies against F protein of RSV

The neutralization activity of the recombinant monoclonal antibodies against F protein of RSV (R3B1h1, R22B1, Nirsevimab, Clesrovimab, and Palivizumab) against RSV/18537 was determined with reference to the protocol of Section 4.4.1. The results showed (Figure 6) that R3B1h1 has the best activity, which is about 18-fold higher than that of Nirsevimab, about 12-fold higher than that of Clesrovimab, and about 545-fold higher than that of Palivizumab, based on the comparison of the IC₅₀ values (Table 4).

**Table 4. Semi-inhibitory concentrations of the monoclonal antibody antibodies against F protein of RSV inhibiting the strain RSV/18537 infection of Hep-2 cells**

| Name of Antibody | IC₅₀(nM) |
|---|---|
| R3B1h1 | 0.007595 |
| R22B1 | 0.1111 |
| Nirsevimab | 0.1392 |
| Clesrovimab | 0.08802 |
| Palivizumab | 4.146 |
| Isotype control antibody | / |

### 4.4.3 Inhibition of the clinically isolated RSV infection of Hep-2 cells by the recombinant monoclonal antibodies against F protein of RSV

The neutralization activity of the recombinant monoclonal antibodies against F protein of RSV (R3B1h1, R22B1, Nirsevimab, Clesrovimab, and Palivizumab) against the clinically isolated RSV was determined with reference to the protocol of Section 4.4.1.

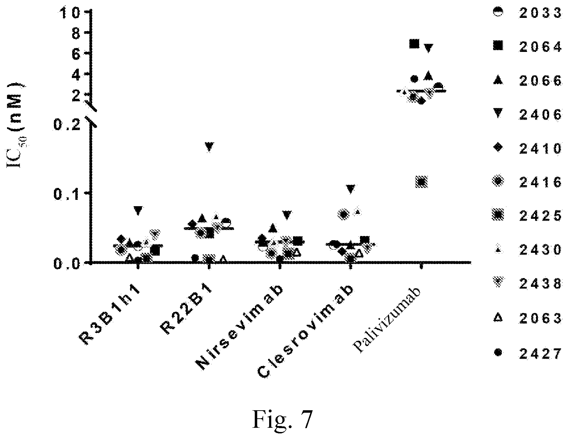

A total of 11 strains isolated from clinical samples taken during the RSV epidemic season in 2021 were obtained from Guangzhou Women and Children's Medical Center. Among them, 9 strains belong to subtype A (being numbered 2033, 2064, 2066, 2406, 2410, 2416, 2425, 2430 and 2438, respectively) and 2 strains belong to subtype B (being numbered 2063 and 2427, respectively). The results showed (Figure 7) that both R3B1h1 and R22B1 can well be able to neutralize RSV clinical isolates.

### Sequence information

**SEQ ID NO:1** 
CGAATAAATACCTGTGACGGAAGATCACTTCGCAGAATAAATAAATCCTGGT
GTCCCTGTTGATACCGGGAAGCCCTGGGCCAACTTTTGGCGAAAATGAGACG
TTGATCGGCACGTAAGAGGTTCCAACTTTCACCATAATGAAATAAGATCACTA
CCGGGCGTATTTTTTGAGTTATCGAGATTTTCA
**SEQ ID NO:2** 
AAATAATGATTTTATTTTGACTGATAGTGACCTGTTCGTTGCAACACATTGATG
AGCAATGCTTTTTTATAATGCCAACTTTGTACAAAAAAGCTGAACGAGAAAC
GTAAAATGATATAAATATCAATATATTAAATTAGATTTTGCATAAAAAACAGAC
TACATAATACTGTAAAACACAACATATCCAGTCACTATGAATCAACTACTTAG
ATGGTATTAGTGACCTGTA
**SEQ ID NO:3** 
GATCAAAGGATCTTCTTGAGATCCTTTTTTTCTGCGCGTAATCTTTTGCCCTGT
AAACGAAAAAACCACCTGGGGAGGTGGTTTGATCGAAGGTTAAGTCAGTTG
GGGAACTGCTTAACCGTGGTAACTGGTTTTCGCAGAGCACAGCAACCAAAT
CTGTCCTTCCAGTGTAGCCGGACTTTGGCGCACACTTCAAGAGCAACCGCGT
GTTTAGCTAAACAAATCCTCTGCGAACTCCCAGTTACCAATGGCTGCTGCCA
GTGGCGTTTTACCGTGCTTTTCCGGGTTGGACTCAAGTGAACAGTTACCGGA
TAAGGCGCAGCAGTCGGGCTGAACGGGGAGTTCTTGCTTACAGCCCAGCTT
GGAGCGAACGACCTACACCGAGCCGAGATACCAGTGTGTGAGCTATGAGAA
AGCGCCACACTTCCCGTAAGGGAGAAAGGCGGAACAGGTATCCGGTAAACG
GCAGGGTCGGAACAGGAGAGCGCAAGAGGGAGCGACCCGCCGGAAACGGT
GGGGATCTTTAAGTCCTGTCGGGTTTCGCCCGTACTGTCAGATTCATGGTTGA
GCCTCACGGCTCCCACAGATGCACCGGAAAAGCGTCTGTTTATGTGAACTCT
GGCAGGAGGGCGGAGCCTATGGAAAAACGCCACCGGCGCGGCCCTGCTGTT
TTGCCTCACATGTTAGTCCCCTGCTTATCCACGGAATCTGTGGGTAACTTTGT
ATGTGTCCGCAGCGC
**SEQ ID NO:4**
   ACAAGTTTGTACAAAAAAGCAGGCT
**SEQ ID NO:5** 
ATGGAGAAAAAAATCACTGGATATACCACCGTTGATATATCCCAATGGCATCG
TAAAGAACATTTTGAGGCATTTCAGTCAGTTGCTCAATGTACCTATAACCAGA
CCGTTCAGCTGGATATTACGGCCTTTTTAAAGACCGTAAAGAAAAATAAGCA
CAAGTTTTATCCGGCCTTTATTCACATTCTTGCCCGCCTGATGAATGCTCATCC
GGAATTCCGTATGGCAATGAAAGACGGTGAGCTGGTGATATGGGATAGTGTT
CACCCTTGTTACACCGTTTTCCATGAGCAAACTGAAACGTTTTCATCGCTCTG
GAGTGAATACCACGACGATTTCCGGCAGTTTCTACACATATATTCGCAAGATG
TGGCGTGTTACGGTGAAAACCTGGCCTATTTCCCTAAAGGGTTTATTGAGAAT
ATGTTTTTCGTCTCAGCCAATCCCTGGGTGAGTTTCACCAGTTTTGATTTAAA
CGTGGCCAATATGGACAACTTCTTCGCCCCCGTTTTCACTATGGGCAAATATT
ATACGCAAGGCGACAAGGTGCTGATGCCGCTGGCGATTCAGGTTCATCATGC
CGTCTTTGATGGCTTCCATGTCGGCAGAATGCTTAATGAATTACAACAGTACT
GCGATGAGTGGCAGGGCGGGGCGTAA
**SEQ ID NO:6** 
TCAGGTGGCACTTTTCGGGGAAATGTGCGCGGAACCCCTATTTGTTTATTTTT
CTAAATACATTCAAATATGTATCCGCTCATGAGACAATAACCCTGATAAATGCT
TCAATAATATTGAAAAAGGAAGAGTATGAGTATTCAACATTTCCGTGTCGCCC
TTATTCCCTTTTTTGCGGCATTTTGCCTTCCTGTTTTTGCTCACCCAGAAACGC
TGGTGAAAGTAAAAGATGCTGAAGATCAGTTGGGTGCACGAGTGGGTTACA
TCGAACTGGATCTCAACAGCGGTAAGATCCTTGAGAGTTTTCGCCCCGAAGA
ACGTTTTCCAATGATGAGCACTTTTAAAGTTCTGCTATGTGGCGCGGTATTAT
CCCGTATTGACGCCGGGCAAGAGCAACTCGGTCGCCGCATACACTATTCTCA
GAATGACTTGGTTGAGTACTCACCAGTCACAGAAAAGCATCTTACGGATGGC
ATGACAGTAAGAGAATTATGCAGTGCTGCCATAACCATGAGTGATAACACTG
CGGCCAACTTACTTCTGACAACGATCGGAGGACCGAAGGAGCTAACCGCTT
TTTTGCACAACATGGGGGATCATGTAACTCGCCTTGATCGTTGGGAACCGGA
GCTGAATGAAGCCATACCAAACGACGAGCGTGACACCACGATGCCTGTAGC
AATGGCAACAACGTTGCGCAAACTATTAACTGGCGAACTACTTACTCTAGCT
TCCCGGCAACAATTAATAGACTGGATGGAGGCGGATAAAGTTGCAGGACCAC
 
TTCTGCGCTCGGCCCTTCCGGCTGGCTGGTTTATTGCTGATAAATCTGGAGCC
GGTGAGCGTGGGTCTCGCGGTATCATTGCAGCACTGGGGCCAGATGGTAAGC
CCTCCCGTATCGTAGTTATCTACACGACGGGGAGTCAGGCAACTATGGATGA
ACGAAATAGACAGATCGCTGAGATAGGTGCCTCACTGATTAAGCATTGGTAA
CTGTCAGACCAAGTTTACTCATATATACTTTAGATTGATTTAAAACTTCATTTT
TAATTTAAAAGGATCTAGGTGAAGATCCTTTTTGATAATCTCATGACCAAAAT
CCCTTAACGTGAGTTTTCGTTCCACTGAGCGTCAGACCCCGTAGAAAAGATC
AAAGGATCTTC
**SEQ ID NO:7** 
CTGGCACGACAGGTTTCCCGACTGGAAAGCGGGCAGTGAGCGCAACGCAAT
TAATGTAAGTTAGCTCACTCATTAGGCACCCCAGGCTTTACACTTTATGCTTC
CGGCTCGTATAATGTGTGGAATTGTGAGCGGATAACAATTTCACACAGGAAA
CAGCTATGACCATGATTACGGATTCACTGGCCGTCGTTTTACAACGTCGTGAC
TGGGAAAACCCTGGCGTTACCCAACTTAATCGCCTTGCAGCACATCCCCCTT
TCGCCAGCTGGCGTAATAGCGAAGAGGCCCGCACCGATCGCCCTTCCCAACA
GTTGCGCAGCCTGAATGGCGAATGGCGCTTTGCCTGGTTTCCGGCACCAGAA
GCGGTGCCGGAAAGCTGGCTGGAGTGCGATCTTCCTGAGGCCGATACTGTCG
TCGTCCCCTCAAACTGGCAGATGCACGGTTACGATGCGCCCATCTACACCAA
CGTGACCTATCCCATTACGGTCAATCCGCCGTTTGTTCCCACGGAGAATCCGA
CGGGTTGTTACTCGCTCACATTTAATGTTGATGAAAGCTGGCTACAGGAAGG
CCAGACGCGAATTATTTTTGATGGCGTCGGGATCTGATCCGGATTTACTAACT
GGAAGAGGCACTAAATGGGAAGAAGGCGAAGTCATGAGCGCCGGGATTTAC
CCCCTAACCTTTATATAAGAAACAATGGATATTACTGCTACAGGGACCCAAGG
ACGGGTAAAGAGTTTGGATTAGGCAGAGACAGGCGAATCGCAATCACTGAA
GCTATACAGGCCAACATTGAGTTATTTTCAGGACACAAACACAAGCCTCTGA
CAGCGAGAATCAACAGTGATAATTCCGTTACGTTACATTCATGGCTTGATCGC
TACGAAAAAATCCTGGCCAGCAGAGGAATCAAGCAGAAGACACTCATAAAT
TACATGAGCAAAATTAAAGCAATAAGGAGGGGTCTGCCTGATGCTCCACTTG
AAGACATCACCACAAAAGAAATTGCGGCAATGCTCAATGGATACATAGACGA
GGGCAAGGCGGCGTCAGCCAAGTTAATCAGATCAACACTGAGCGATGCATTC
CGAGAGGCAATAGCTGAAGGCCATATAACAACAAACCATGTCGCTGCCACTC
GCGCAGCAAAATCAGAGGTAAGGAGATCAAGACTTACGGCTGACGAATACC
TGAAAATTTATCAAGCAGCAGAATCATCACCATGTTGGCTCAGACTTGCAAT
GGAACTGGCTGTTGTTACCGGGCAACGAGTTGGTGATTTATGCGAAATGAAG
TGGTCTGATATCGTAGATGGATATCTTTATGTCGAGCAAAGCAAAACAGGCGT
AAAAATTGCCATCCCAACAGCATTGCATATTGATGCTCTCGGAATATCAATGA
AGGAAACACTTGATAAATGCAAAGAGATTCTTGGCGGAGAAACCATAATTGC
ATCTACTCGTCGCGAACCGCTTTCATCCGGCACAGTATCAAGGTATTTTATGC
GCGCACGAAAAGCATCAGGTCTTTCCTTCGAAGGGGATCCGCCTACCTTTCA
CGAGTTGCGCAGTTTGTCTGCAAGACTCTATGAGAAGCAGATAAGCGATAAG
TTTGCTCAACATCTTCTCGGGCATAAGTCGGACACCATGGCATCACAGTATCG
TGATGACAGAGGCAGGGAGTGGGACAAAATTGAAATCAAATAACTAGCATA
ACCCCTTGGGGCCTCTAAACGGGTCTTGAGGGGTTTTTTG
**SEQ ID NO:8** 
AAATAATGATTTTATTTTGACTGATAGTGACCTGTTCGTTGCAACACATTGATG
AGCAATGCTTTTTTATAATGCCAACTTTGTACAAAAAAGCAGGCT
**SEQ ID NO:9** 
ACAAGTTTGTACAAAAAAGCTGAACGAGAAACGTAAAATGATATAAATATCA
ATATATTAAATTAGATTTTGCATAAAAAACAGACTACATAATACTGTAAAACAC
 AACATATCCAGTCACTATGAATCAACTACTTAGATGGTATTAGTGACCTGTA
**SEQ ID NO:10** 
MELLILKANAITTILTAVTFCFASGQNITEEFYQSTCSAVSKGYLSALRTGWYTSV
ITIELSNIKENKCNGTDAKVKLIKQELDKYKNAVTELQLLMQSTPPTNNRARRE
LPRFMNYTLNNAKKTNVTLSKKRKRRFLGFLLGVGSAIASGVAVSKVLHLEGE
VNKIKSALLSTNKAVVSLSNGVSVLTSKVLDLKNYIDKQLLPIVNKQSCSISNIE
TVIEFQQKNNRLLEITREFSVNAGVTTPVSTYMLTNSELLSLINDMPITNDQKKL
MSNNVQIVRQQSYSIMSIIKEEVLAYVVQLPLYGVIDTPCWKLHTSPLCTTNTKE
GSNICLTRTDRGWYCDNAGSVSFFPQAETCKVQSNRVFCDTMNSLTLPSEINLC
NVDIFNPKYDCKIMTSKTDVSSSVITSLGAIVSCYGKTKCTASNKNRGIIKTFSN
GCDYVSNKGMDTVSVGNTLYYVNKQEGKSLYVKGEPIINFYDPLVFPSDEFDA
SISQVNEKINQSLAFIRKSDELLHNVNAGKSTTNIMITTIIIVIIVILLSLIAVGLLLY
CKARSTPVTLSKDQLSGINNIAFSN
**SEQ ID NO:11** 
MELLIHRSSAIFLTLAVNALYLTSSQNITEEFYQSTCSAVSRGYFSALRTGWYTSV
ITIELSNIKETKCNGTDTKVKLIKQELDKYKNAVTELQLLMQNTPAANNRARRE
APQYMNYTINTTKNLNVSISKKRKRRFLGFLLGVGSAIASGIAVSKVLHLEGEV
NKIKNALLSTNKAVVSLSNGVSVLTSKVLDLKNYINNRLLPIVNQQSCRISNIET
VIEFQQMNSRLLEITREFSVNAGVTTPLSTYMLTNSELLSLINDMPITNDQKKLM
SSNVQIVRQQSYSIMSIIKEEVLAYVVQLPIYGVIDTPCWKLHTSPLCTTNIKEGS
NICLTRTDRGWYCDNAGSVSFFPQADTCKVQSNRVFCDTMNSLTLPSEVSLCNT
DIFNSKYDCKIMTSKTDISSSVITSLGAIVSCYGKTKCTASNKNRGIIKTFSNGCD
YVSNKGVDTVSVGNTLYYVNKLEGKNLYVKGEPIINYYDPLVFPSDEFDASISQ
VNEKINQSLAFIRRSDELLHNVNTGKSTTNIMITTIIIVIIVVLLSLIAIGLLLYCKA
KNTPVTLSKDQLSGINNIAFSK
**SEQ ID NO:12** 
MELLILKANAITTILTAVTFCFASGQNITEEFYQSTCSAVSKGYLSALRTGWYTSV
ITIELSNIKENKCNGTDAKVKLIKQELDKYKNAVTELQLLMQSTPPTNNRARRE
LPRFMNYTLNNAKKTNVTLSKKRKRRFLGFLLGVGSAIASGVAVCKVLHLEGE
VNKIKSALLSTNKAVVSLSNGVSVLTFKVLDLKNYIDKQLLPILNKQSCSISNIET
VIEFQQKNNRLLEITREFSVNAGVTTPVSTYMLTNSELLSLINDMPITNDQKKL
MSNNVQIVRQQSYSIMCIIKEEVLAYVVQLPLYGVIDTPCWKLHTSPLCTTNTK
EGSNICLTRTDRGWYCDNAGSVSFFPQAETCKVQSNRVFCDTMNSLTLPSEINL
CNVDIFNPKYDCKIMTSKTDVSSSVITSLGAIVSCYGKTKCTASNKNRGIIKTFS
NGCDYVSNKGMDTVSVGNTLYYVNKQEGKSLYVKGEPIINFYDPLVFPSDEFD
ASISQVNEKINQSLAFIRKSDELLSAIGGYIPEAPRDGQAYVRKDGEWVLLSTFL
**SEQ ID NO:13** 
MELLIHRSSAIFLTLAVNALYLTSSQNITEEFYQSTCSAVSRGYFSALRTGWYTSV
ITIELSNIKETKCNGTDTKVKLIKQELDKYKNAVTELQLLMQNTPAANNRARRE
APQYMNYTINTTKNLNVSISKKRKRRFLGFLLGVGSAIASGIAVCKVLHLEGEV
NKIKNALLSTNKAVVSLSNGVSVLTFKVLDLKNYINNRLLPILNQQSCRISNIET
VIEFQQMNSRLLEITREFSVNAGVTTPLSTYMLTNSELLSLINDMPITNDQKKLM
SSNVQIVRQQSYSIMCIIKEEVLAYVVQLPIYGVIDTPCWKLHTSPLCTTNIKEGS
NICLTRTDRGWYCDNAGSVSFFPQADTCKVQSNRVFCDTMNSLTLPSEVSLCNT
DIFNSKYDCKIMTSKTDISSSVITSLGAIVSCYGKTKCTASNKNRGIIKTFSNGCD
YVSNKGVDTVSVGNTLYYVNKLEGKNLYVKGEPIINYYDPLVFPSDEFDASISQ
VNEKINQSLAFIRRSDELLSAIGGYIPEAPRDGQAYVRKDGEWVLLSTFL
**SEQ ID NO:14**
   HHHHHH
**SEQ ID NO:15** 
ASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKRVEPKSCDKTHT
CPPCPAPELLGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAP
IEKTISKAKGQPREPQVYTLPPSREEMTKNQVSLTCLVKGFYPSDIAVEWESNGQ
PENNYKTTPPVLDSDGSFFLYSKLTVDKSRWQQGNVFSCSVMHEALHNHYTQK
SLSLSPGK
**SEQ ID NO:16** 
ASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKRVEPKSCDKTHT
CPPCPAPELLGGPSVFLFPPKPKDTLYITREPEVTCVVVDVSHEDPEVKFNWYV
DGVEVHNAKTKPREEQYNSTYRVVSVLTVLHQDWLNGKEYKCKVSNKALPAP
IEKTISKAKGQPREPQVYTLPPSREEMTKNQVSLTCLVKGFYPSDIAVEWESNGQ
PENNYKTTPPVLDSDGSFFLYSKLTVDKSRWQQGNVFSCSVMHEALHNHYTQK
SLSLSPGK
**SEQ ID NO:17** 
AKTTAPSVYPLAPVCGDTTGSSVTLGCLVKGYFPEPVTLTWNSGSLSSGVHTFP
AVLQSDLYTLSSSVTVTSSTWPSQSITCNVAHPASSTKVDKKIEPRGPTIKPCPPC
KCPAPNLLGGPSVFIFPPKIKDVLMISLSPIVTCVVVDVSEDDPDVQISWFVNNV
EVHTAQTQTHREDYNSTLRVVSALPIQHQDWMSGKEFKCKVNNKDLPAPIERTI
SKPKGSVRAPQVYVLPPPEEEMTKKQVTLTCMVTDFMPEDIYVEWTNNGKTEL
NYKNTEPVLDSDGSYFMYSKLRVEKKNWVERNSYSCSVVHEGLHNHHTTKSF
SRTPGK
**SEQ ID NO:18**
**SEQ ID NO:19** 
GQPKAAPSVTLFPPSSEELQANKATLVCLISDFYPGAVTVAWKADSSPVKAGVE
TTTPSKQSNNKYAASSYLSLTPEQWKSHRSYSCQVTHEGSTVEKTVAPTECS
**SEQ ID NO:20** 
RADAAPTVSIFPPSSEQLTSGGASVVCFLNNFYPKDINVKWKIDGSERQNGVLN
SWTDQDSKDSTYSMSSTLTLTKDEYERHNSYTCEATHKTSTSPIVKSFNRNEC
**SEQ ID NO:21** 
GQPKSSPSVTLFPPSSEELETNKATLVCTITDFYPGVVTVDWKVDGTPVTQGME
TTQPSKQSNNKYMASSYLTLTARAWERHSSYSCQVTHEGHTVEKSLSRADCS
**SEQ ID NO:22** 
QVTLRESGPALVKPTQTLTLTCTFSGFSLSTSGMSVGWIRQPPGKALEWLADIW
WDDKKDYNPSLKSRLTISKDTSKNQVVLKVTNMDPADTATYYCARSMITNWY
FDVWGAGTTVTVSS
**SEQ ID NO:23** 
DIQMTQSPSTLSASVGDRVTITCKCQLSVGYMHWYQQKPGKAPKLWIYDTSKL
ASGVPSRFSGSGSGTEFTLTISSLQPDDFATYYCFQGSGYPFTFGGGTKLEIK
**SEQ ID NO:24**
**SEQ ID NO:25**
**SEQ ID NO:26** 
EVQLVESGGGLVRPGRSLRLSCTVSGFSFDDSAMSWVRQAPGKGLEWISFIKSK
TYGGTKEYAASVKGRFTISRDDSKNIAYLQMNSLKTEDTAVYYCTRGAPYGGN
SDYYYGLDVWGQGTTVTVSS
**SEQ ID NO:27** 
DIQMTQSPSSLSASVGDRVTITCRTSQDVRGALAWYQQKPGKAPKLLIFDASSL
ETGVPSRFSGSGSGTVFTLTISSLQPEDFAAYYCQQFLDFPFTFGQGTRLEIK
**SEQ ID NO:28** 
QVQLVQSGAEVKKPGSSVKVSCKASGGLLEDYIINWVRQAPGQGLEWMGGIIP
VLGTVHYGPKFQGRVTITADESTSTAYMELSSLRSEDTAVYYCATETAIVVTESY
RPHFFDNWGQGTLVTVSS
**SEQ ID NO:29** 
DIQMTQSPSSLSAsVGDRVTITCQASQDIVNYLNWYQQKPGKAPKLLIYVASNL
ETGVPSRFSGSGSGTDFtfTISSLQPEDiATYYCQEFAGLALNFGGGTKVEIK
**SEQ ID NO:30** 
QVQLVQSGAEVKKPGSSVMVSCQASGDPLQNYIINWVRQAPGQGPEWMGAIIP
ALGSAHYGPKFQGRVTITADESTDTAYMELSSLRSEDTAMYYCATETAVIISESY
LPHFFDNWGQGTLVTVSS
**SEQ ID NO:31** 
DIQMTQSPSSLSAAVGDRVTITCQASQDIVNYLNWYQQKPGKAPKLLIYVASNL
ETGVPSRFSGSGSGTDFSLTISSLQPEDVATYYCQEYDSLALAFGGGTKVEIK
**SEQ ID NO:32**
   DYIIN
**SEQ ID NO:33**
   GIIPVLGTVHYGPKFQG
**SEQ ID NO:34**
   ETAIVVTESYRPHFFDN
**SEQ ID NO:35**
   QASQDIVNYLN
**SEQ ID NO:36**
   VASNLET
**SEQ ID NO:37**
   QEFAGLALN
**SEQ ID NO:38**
   NYIIN
**SEQ ID NO:39**
   AIIPALGSAHYGPKFQG
**SEQ ID NO:40**
   ETAVIISESYLPHFFDN
**SEQ ID** NO:41
   QASQDIVNYLN
**SEQ ID NO:42**
   VASNLET
**SEQ ID NO:43**
   QEYDSLALA
**SEQ ID NO:44** 
TTGAGATCCTTTTTTTCTGCGCGTAATCTGCTGCTTGCAAACAAAAAAACCA
CCGCTACCAGCGGTGGTTTGTTTGCCGGATCAAGAGCTACCAACTCTTTTTC
CGAAGGTAACTGGCTTCAGCAGAGCGCAGATACCAAATACTGTCCTTCTAGT
GTAGCCGTAGTTAGGCCACCACTTCAAGAACTCTGTAGCACCGCCTACATAC
CTCGCTCTGCTAATCCTGTTACCAGTGGCTGCTGCCAGTGGCGATAAGTCGT
GTCTTACCGGGTTGGACTCAAGACGATAGTTACCGGATAAGGCGCAGCGGTC
GGGCTGAACGGGGGGTTCGTGCACACAGCCCAGCTTGGAGCGAACGACCTA
CACCGAACTGAGATACCTACAGCGTGAGCTATGAGAAAGCGCCACGCTTCCC
GAAGGGAGAAAGGCGGACAGGTATCCGGTAAGCGGCAGGGTCGGAACAGG
AGAGCGCACGAGGGAGCTTCCAGGGGGAAACGCCTGGTATCTTTATAGTCCT
GTCGGGTTTCGCCACCTCTGACTTGAGCGTCGATTTTTGTGATGCTCGTCAG
GGGGGCGGAGCCTATGGAAA
**SEQ ID NO:45** 
CGCGCCCTGTAGCGGCGCATTAAGCGCGGCGGGTGTGGTGGTTACGCGCAG
CGTGACCGCTACACTTGCCAGCGCCCTAGCGCCCGCTCCTTTCGCTTTCTTCC
CTTCCTTTCTCGCCACGTTCGCCGGCTTTCCCCGTCAAGCTCTAAATCGGGG
GCTCCCTTTAGGGTTCCGATTTAGTGCTTTACGGCACCTCGACCCCAAAAAA
CTTGATTAGGGTGATGGTTCACGTAGTGGGCCATCGCCCTGATAGACGGTTTT
TCGCCCTTTGACGTTGGAGTCCACGTTCTTTAATAGTGGACTCTTGTTCCAAA
CTGGAACAACACTCAACCCTATCTCGGTCTATTCTTTTGATTTATAAGGGATTT
TGCCGATTTCGGCCTATTGGTTAAAAAATGAGCTGATTTAACAAAAATTTAAC
GCGAATTTTAACAAAATATTAACGCTTACAATTT
**SEQ ID NO:46**

GGTGGCGGCTCCGGTTCCGGTGATTTTGATTATGAAAAAATGGCAAACGCTA
ATAAGGGGGCTATGACCGAAAATGCCGATGAAAACGCGCTACAGTCTGACG
CTAAAGGCAAACTTGATTCTGTCGCTACTGATTACGGTGCTGCTATCGATGGT
TTCATTGGTGACGTTTCCGGCCTTGCTAATGGTAATGGTGCTACTGGTGATTT
TGCTGGCTCTAATTCCCAAATGGCTCAAGTCGGTGACGGTGATAATTCACCTT
TAATGAATAATTTCCGTCAATATTTACCTTCCCTCCCTCAATCGGTTGAATGTC
GCCCTTTTGTCTTTGGCGCTGGTAAACCATATGAATTTTCTATTGATTGTGACA
AAATAAACTTATTCCGTGGTGTCTTTGCGTTTCTTTTATATGTTGCCACCTTTA
TGTATGTATTTTCTACGTTTGCTAACATACTGCGTAATAAGGAGTCTTGATAA

### Reference

1. JAMA Pediatr. 2016 Mar;170(3):267-87.
2. Lancet Glob Health. 2017 Oct;5(10): e984-e991.
3. J Gen Virol. 2017 Dec;98(12):2912-2913.
4. J Gen Virol. 1985 Oct;66 (Pt 10):2111-24.
5. J Virol. 2008 Mar;82(5):2040-55.
6. J Virol. 1987 Oct;61(10):3163-6.
7. Proc Natl Acad Sci U S A. 1984 Dec;81(24):7683-7.
8. Virus Res. 2000 Jun;68(1):25-33.
9. Virology. 1993 Nov;197(1):1-11.
10. Gene. 1994 Dec 30;151(1-2):109-13.
11. Kabat, "Sequences of Proteins of Immunological Interest" , National Institutes of Health, Bethesda, Md. (1991).
12. A1-Lazikani, et al., J. Mol. Biol. 273:927-948 (1997).
13. Martin, et al., Proc. Natl. Acad. Sci.USA86:9268-9272 (1989).
14. Annu Rev Biochem. 1989; 58:913-49.
15. Science. 2013 Nov 1;342(6158):592-8.

## Claims

1. An antibody against respiratory syncytial virus (RSV) comprising a heavy chain variable region comprising the amino acid sequences of HCDR1, HCDR2 and HCDR3, and a light chain variable region comprising the amino acid sequences of LCDR1, LCDR2 and LCDR3, wherein
the amino acid sequence of HCDR1 is set forth in SEQ ID NO:32, the amino acid sequence of HCDR2 is set forth in SEQ ID NO:33, the amino acid sequence of HCDR3 is set forth in SEQ ID NO:34, the amino acid sequence of LCDR1 is set forth in SEQ ID NO:35, the amino acid sequence of LCDR2 is set forth in SEQ ID NO:36, and the amino acid sequence of LCDR3 is set forth in SEQ ID NO:37; or
the amino acid sequence of HCDR1 is set forth in SEQ ID NO:38, the amino acid sequence of HCDR2 is set forth in SEQ ID NO:39, the amino acid sequence of HCDR3 is set forth in SEQ ID NO:40, the amino acid sequence of LCDR1 is set forth in SEQ ID NO:41, the amino acid sequence of LCDR2 is set forth in SEQ ID NO:42, and the amino acid sequence of LCDR3 is set forth in SEQ ID NO:43;
wherein the amino acid sequences of HCDRs and LCDRs are defined according to Kabat.

2. The antibody according to claim 1, wherein the amino acid sequence of the heavy chain variable region of the antibody is set forth in SEQ ID NO: 28 or 30.

3. The antibody according to claim 1, wherein the amino acid sequence of the light chain variable region of the antibody is set forth in SEQ ID NO: 29 or 31.

4. The antibody according to any one of claims 1-3, wherein
the amino acid sequence of the heavy chain variable region of the antibody is set forth in SEQ ID NO: 28, and the amino acid sequence of the light chain variable region of the antibody is set forth in SEQ ID NO: 29; or
the amino acid sequence of the heavy chain variable region of the antibody is set forth in SEQ ID NO: 30, and the amino acid sequence of the light chain variable region of the antibody is set forth in SEQ ID NO: 31.

5. An antibody against respiratory syncytial virus (RSV), wherein the amino acid sequence of the heavy chain variable region of the antibody has at least 90% identity to the amino acid sequence as set forth in SEQ ID NO: 28 or 30, and the amino acid sequence of the light chain variable region of the antibody has at least 90% identity to the amino acid sequence as set forth in SEQ ID NO: 29 or 31.

6. The antibody according to any one of claims 1-5, wherein
the antibody is a neutralizing antibody; and/or
the antibody is capable of binding to F protein of human respiratory syncytial virus (RSV); preferably, the antibody is capable of binding a recombinant F protein of human RSV as set forth in SEQ ID NO: 12 or 13; and/or
the antibody is a Fab fragment, a complete antibody, a F(ab')₂ fragment, or a single chain Fv fragment (scFv); preferably, the antibody is a Fab fragment; and/or
the antibody is a monoclonal antibody.

7. The antibody according to any one of claims 1-6, wherein
the antibody comprises a heavy chain constant region of an IgG1 subtype, an IgG2 subtype, or an IgG4 subtype; preferably, the heavy chain constant region comprises a sequence of the Fc fragment of the heavy chain constant region of the IgG1 subtype, and the amino acids at positions 252, 254 and 256 of the sequence of the Fc fragment are Y, T and E, respectively, wherein the amino acid positions of the constant region of the antibody is determined according to EU numbering; and/or
the antibody comprises a light chain constant region of a kappa subtype or a lambda subtype.

8. A nucleic acid molecule encoding the antibody according to any one of claims 1-7.

9. A pharmaceutical composition comprising the antibody according to any one of claims 1-7 and a pharmaceutically acceptable excipient, diluent or carrier.

10. The pharmaceutical composition according to claim 9, for use in the prevention or treatment of an RSV-associated disease; preferably, the RSV-associated disease is a respiratory tract infection such as bronchiolitis and pneumonia.

11. Use of the antibody according to any one of claims 1-7, the nucleic acid molecule according to claim 8, or the pharmaceutical composition according to claim 9 or 10 in the manufacture of a medicament for the prevention or treatment of an RSV-associated disease; preferably, the RSV-associated disease is a respiratory tract infection such as bronchiolitis and pneumonia.

12. A method of preventing or treating an RSV-associated disease, comprising administering to a subject in need thereof the antibody according to any one of claims 1-7, the nucleic acid molecule according to claim 8, or the pharmaceutical composition according to claim 9 or 10; preferably, the RSV-associated disease is a respiratory tract infection such as bronchiolitis and pneumonia.
